# EUROPEAN PATENT APPLICATION

(11) **EP 4 442 320 A2**
(43) Date of publication of application: **09.10.2024**
(21) Application number: 24193030.4
(22) Date of filing: 11.02.2019
(51) Int. Cl.: A61P 31/14

(54) **METHODS OF INHIBITING INFLAMMASOME ACTIVATION**

(30) Priority: 12.02.2018 US 201862629435 P; 02.03.2018 US 201862637460 P
(62) Divisional of application: 19711181.8
(71) Applicant: TRUSTEES OF TUFTS COLLEGE, Medford, MA 02155 (US)
(72) Inventor: KUMAR-SINGH, Rajendra, Weston, MA 02493 (US); KUMAR, Binit, Hillsborough Township, NJ 08844 (US); CASHMAN, Siobhan M., Concord, MA 01742 (US)
(74) Representative: HGF

(57) **Abstract**

Methods and kits are provided for inhibiting inflammasome activation in cells of a subject or an inflammation-affected eye in a subject by administering to the subject a composition including a nucleotide sequence encoding a membrane independent CD59 protein operably linked to a promoter for expression and secretion of the membrane independent CD59 protein in the cells or the inflammation-affected eye thereby or a soluble CD59 protein the composition inhibiting inflammasome activation.

## Description

### Related Applications

This application claims the benefit of priority to U.S. Provisional Patent Application No. 62/629,435, filed February 12, 2018; and U.S. Provisional Patent Application No. 62/637,460, filed March 2, 2018.

### Government Support

This invention was made with government support under grant number EY021805 awarded by the National Institutes of Health. The government has certain rights in the invention.

### Background

Complement is a critical component of the innate immune system and its role has been described in various inflammatory diseases including autoimmune diseases, cancer, ischemia/reperfusion injury, among others (Morgan, B. P., et al. (2015) Nat. Rev. Drug Discov. 14, 857-877). A role for complement has also been previously described in experimental model of autoimmune uveitis (EAU) (An, F., et al. (2009) Invest. Ophthalmol. Vis. Sci. 50, 3778-3782; Copland, D. A., et al. (2010) Clin. Exp. Immunol. 159, 303-314; Read, R. W., et al. (2006) Exp. Eye Res. 82, 389-394; Zhang, L., et al. (2016) J. Leukoc. Biol. 99, 447-454). Little is known about the mechanisms that connect activation of complement with T cell mediated pathology in EAU. Activation of complement terminates with the assembly of the membrane attack complex (MAC) on the membrane of cells, resulting in the formation of a pore. At sublytic level the formation of the pore facilitates ion exchange across the plasma membrane and the release of cytokines. As a result, deposition of MAC leads to cell lysis. The role of MAC in the pathophysiology of EAU is as yet undetermined. Entry of Ca2+ into the cell following deposition of MAC is known to activate the NLRP3 inflammasome in primary lung human epithelial cells (Triantafilou, K., et al. (2013) J. Cell Sci. 126, 2903-2913).

The NLRP3 inflammasome has been implicated in various inflammatory disorders including age-related macular degeneration, cardiomyopathy, arthritis, chronic kidney disease, neurodegenerative diseases etc. (Amin, J., et al. (2017) Brain pathol. 27, 192-204). Administration of lipopolysaccharide (LPS) in mice leads to MAC associated IL-1β maturation (Laudisi, F., et al. (2013) J. Immunol. 191, 1006-1010). Although the inflammasome is highly regulated by complement and is necessary during resolution of tissue injury or disease, the unregulated inflammasome can lead to severe inflammation and damage to host tissues (Latz, E., et al. (2013) Nat. Rev. Immunol.13, 397-411). Activation of the NLRP3 inflammasome is controlled by a two-step process that requires an initial priming signal which involves increased expression of the protein NLRP3 and a subsequent activation signal required for formation of the inflammasome protein complex that results in caspase-1-mediated cleavage of pro-IL-1β into mature IL-1β (Broz, P., et al. (2016) Nat. Rev. Immunol. 16, 407-420). Unregulated IL-1β expression can lead to the development of autoimmune and auto-inflammatory diseases including Behcet's disease, Vogt-Koyanagi-Harada disease, rheumatic diseases, autoimmune thyroid disease, insulin-dependent diabetes mellitus, gout, familial Mediterranean fever and cryopyrin-associated periodic syndromes (Dinarello, C. A., et al. (2013) Semin. Immunol. 25, 469-484; Gabay, C., et al. (2010) Nat. Rev. Rheumatol. 6, 232-241; Jesus, A. A., et al. (2014) Annu. Rev. Med. 65, 223-244). Further, it has been established that IL-1β is actively secreted in the retina by myeloid cells, indicating a potential pathogenic role of II,-1R signaling in EAU (Wan, C. K., et al. (2016) J. Immunol. 196, 543-546). However, knowledge of detailed mechanisms of how IL-1β is regulated in EAU remains elusive.

Uveitis is a chronic ocular inflammatory disorder of the uveal and retinal layers of the eye responsible for about 10% to about 15% of total blindness in the U.S (Durrani, O. M., et al. (2004) Ophthalmologica 218, 223-236). Uveitis is classified as infectious if an external biological agent triggers an immune response or non-infectious if an autoimmune reaction is triggered. Clinical care for uveitis has been by use of corticosteroids, immunosuppressive drugs or monoclonal antibodies, however these approaches have limited success and are associated with significant side effects (Knickelbein, J. E., et al. (2017) Handb. Exp. Pharmacol. 242, 231-268; Prete, M., et al. (2016) Clin. Exp. Med. 16, 125-136). Thus, there is an unmet clinical need to develop efficacious therapies for uveitis.

### Summary

Described herein are methods, compositions, and kits for treating individuals afflicted with inflammasome related disorders with a membrane independent (soluble) CD59 protein. The membrane independent CD59 protein can be expressed from an administered nucleic acid or from direct administration of the protein. Contemplated are nucleic acids encoding a membrane independent CD59 protein or a composition thereof for use in treating an inflammasome related disorder. Also contemplated is a membrane independent CD59 protein or composition thereof for use in treating an inflammasome related disorder. In certain embodiments, the inflammasome related disorder is in the eye of a subject. In certain embodiments, the inflammasome related disorder comprises a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis. In certain embodiments, the inflammasome related disorder comprises a uveitis. The proteins or nucleic acids encoding the soluble/membrane independent CD59 can be administered to a subject that has displayed positive results for expression or activity of at least one inflammasome activity marker. In certain embodiments the positive results are in an eye of the subject. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3).

An aspect of the invention herein provides a method for inhibiting inflammasome activation in cells of an inflammation-affected eye in a subject, the method including, administering to the subject a composition comprising a nucleotide sequence encoding a membrane independent CD59 protein operably linked to a promoter for expression and secretion of the membrane independent CD59 protein in the cells of the inflammation-affected eye, the composition inhibiting inflammasome activation. The term "membrane independent" as used herein refers to a CD59 amino acid sequence that lacks a GPI anchor or has a modified GPI anchor that lacks function and ability to bind to a cell membrane or a cell-membrane-associated structure such as a membrane-bound protein. An embodiment of the method provides that the membrane independent CD59 inhibits inflammasome activation independent of function of MAC.

In an embodiment of the method, the composition inhibits inflammasome activation independent of function of Membrane Attack Complex (MAC). In an embodiment of the method, the subject has at least one condition selected from: a uveitis, an allergic conjunctivitis, a blepharitis, a chronic conjunctivitis, an episcleritis, a keratitis, a retinitis, a diabetic retinopathy, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis, a Vogt-Koyanagi-Harada Disease, a psoriasis, an immune hemolytic anemia, a thrombocytopenic purpura, an Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis.

In an embodiment of the method, the uveitis is at least one selected from: anterior uveitis, intermediate uveitis, posterior uveitis, and panuveitis. An embodiment of the method further includes obtaining a sample from the subject. In an embodiment of the method, the sample is at least one selected from: tears, blood, urine, eye discharge, phlegm, and mucus.

An embodiment of the method further includes before administering, measuring in the eye at least one of a retinal function of the eye and an inflammasome activity marker. An embodiment of the method further includes after administering, measuring in the eye at least one of a retinal function of the eye and an inflammasome activity marker.
In an embodiment of the method, the inflammasome activity marker is at least one selected from:
caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In an embodiment of the method, the inflammasome activity marker is selected from: apoptosis-associated speck-like protein containing a CARD (PYCARD/ASC), or a NACHT, LRR and PYD domains-containing protein (NALP). In an embodiment of the method, the NALP is NACHT, LRR and PYD domains-containing protein 3 (NLRP3).

In an embodiment of the method, measuring the retinal function or the inflammasome activity marker further includes performing at least one procedure selected from: an eye exam, an Optical Coherence Tomography (OCT), a Conjunctival Impression Cytology (CIT), an RTPCR, an ELISA and a PCR.

An embodiment of the method further includes administering the composition in a dosage sufficient to treat the uveitis. An embodiment of the method further includes prior to administering, engineering the nucleotide sequence in a viral vector. An embodiment of the method further includes administering the nucleotide sequence as a naked nucleic acid.

In an embodiment of the method, the viral vector is a genetically engineered genome of at least one virus selected from the group consisting of: an adenovirus, an adeno-associated virus, a herpesvirus, and a lentivirus. In an embodiment of the method, the lentivirus is a retrovirus.

An embodiment of the method further includes engineering the membrane independent CD59 protein to have at least one mutation resulting in loss of function of a glycosyl phosphatidyl inositol (GPI) anchoring domain of the translated CD59 protein. An embodiment of the method further includes engineering nucleotide sequence encoding the membrane independent CD59 protein by deleting nucleotides encoding the region of the glycosyl phosphatidyl inositol (GPI) anchoring domain.

In an embodiment of the method, administering further includes injecting the composition ocularly. In an embodiment of the method, administering further includes applying the composition topically. In an embodiment of the method, the ocular injection is selected from the group consisting of subretinal injection, vitreous injection, intra-ocular injection, subconjunctival injection, and subtenon injection. In an embodiment of the method, ocular injecting further includes administering to an external layer of the eye.

An embodiment of the method further includes administering an additional therapeutic agent to the eye. In some embodiments of the method, the additional therapeutic agent is at least one selected from the group consisting of: anti-tumor, antiviral, antibacterial, anti-mycobacterial, anti-fungal, anti-proliferative and anti-apoptotic. In some embodiments of the method, the additional therapeutic agent is selected from the group consisting of: a growth factor, an anti-inflammatory agent, a vasopressor agent, a collagenase inhibitor, a steroid, a matrix metalloproteinase inhibitor, an ascorbate, an angiotensin, a calreticulin, a tetracycline, a fibronectin, a collagen, a thrombospondin, a transforming growth factors (TGF), a keratinocyte growth factor (KGF), a fibroblast growth factor (FGF), an insulin-like growth factors (IGFs), an IGF binding protein (IGFBP), an epidermal growth factor (EGF), a platelet derived growth factor (PDGF), a neu differentiation factor (NDF), a hepatocyte growth factor (HGF), a vascular endothelial growth factor (VEGF), a heparin-binding EGF (HBEGF), a thrombospondin, a von Willebrand Factor-C, a heparin, a heparin sulfate, and a hyaluronic acid.

An aspect of the invention herein provides a kit for inhibiting an activity of an inflammasome in an inflammation-affected eye in a subject, the kit including: a pharmaceutical composition including a membrane-independent CD59 protein and/or a nucleotide sequence encoding the CD59 protein, such that the composition is in a dosage sufficient to inhibit the inflammasome in the inflammation-affected eye in the subject; instructions for use; and, a container.

An aspect of the invention herein provides a kit for treating uveitis in a subject including: a pharmaceutical composition comprising a membrane-independent CD59 protein and/or a nucleotide sequence encoding the CD59 protein, such that the composition is in a dosage sufficient to treat the uveitis in the subject; instructions for use; and, a container.

An aspect of the invention herein provides a method for inhibiting inflammasome activation in cells of an inflammation-affected eye in a subject, the method comprising: administering to the subject a composition comprising a nucleotide sequence encoding a membrane independent CD59 protein operably linked to a promoter for expression and secretion of the membrane independent CD59 protein in the cells of the inflammation-affected eye, wherein the composition inhibits inflammasome activation, wherein the subject has displayed positive results for expression or activity of at least one inflammasome activity marker in the inflammation-affected eye of the subject. In certain embodiments of the method, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments of the method, the inflammasome activity marker is selected from: apoptosis-associated speck-like protein containing a CARD (PYCARD/ASC), or a NACHT, LRR and PYD domains-containing protein (NALP). In certain embodiments of the method, the NALP is NACHT, LRR and PYD domains-containing protein 3 (NLRP3). In certain embodiments of the method, the method further comprises measuring in the eye an inflammasome activity marker after the administering of the composition comprising the nucleotide sequence encoding the membrane independent CD59 protein operably linked to the promoter for the expression and secretion of the membrane independent CD59 protein. In certain embodiments of the method, the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis. In certain embodiments, the subject has been diagnosed with or is suspected of being afflicted with a uveitis. The positive results for expression or activity of at least one inflammasome activity marker in the inflammation-affected eye of the subject described herein refers to elevated expression or activity of at least one inflammasome activity marker in comparison to expression or activity of at least one inflammasome activity marker in the eye of an individual not diagnosed or afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a polyarteritis nodosa, a Wegener's granulomatosis a Vogt-Koyanagi-Harada Disease, a sympathetic ophthalmia, or a sarcoidosis. Alternatively, the expression or activity of at least one inflammasome activity marker in the inflammation-affected eye of the subject is determined by a histological score.

A certain aspect described herein, is a method for inhibiting inflammasome activation in the cells of an inflammation-affected subject, the method comprising: (a) administering to the subject a composition comprising a nucleotide sequence encoding a membrane independent CD59 protein operably linked to a promoter for expression and secretion of the membrane independent CD59 protein in the cells of the inflammation-affected subject; or (b) administering to the subject a composition comprising a soluble CD59 protein, wherein the composition inhibits inflammasome activation. In certain embodiments, the composition inhibits inflammasome activation independent of function of Membrane Attack Complex (MAC). In certain embodiments, the subject has at least one inflammasome associated condition selected from: Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis. In certain embodiments, the method further comprises obtaining a sample from the subject. In certain embodiments, the sample is at least one selected from: blood, plasma, serum, peripheral blood mononuclear cells, cerebrospinal fluid, or urine. In certain embodiments, the method further comprises, before administering, measuring in the subject an inflammasome activity marker. In certain embodiments, the method further comprises, after administering, measuring in the subject an inflammasome activity marker. In certain embodiments, the inflammasome activity marker is at least one selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the method further comprises administering the composition in a dosage sufficient to treat the inflammasome associated condition. In certain embodiments, the nucleotide sequence encoding a membrane independent CD59 protein operably linked to a promoter for expression and secretion of the membrane independent CD59 protein is a genetically engineered genome of at least one virus selected from the group consisting of: an adenovirus, an adeno-associated virus, a herpesvirus, and a lentivirus. In certain embodiments, the lentivirus is a retrovirus. In certain embodiments, the administering further comprises injecting the composition intravenously. In certain embodiments, the method further comprises applying the composition topically. In certain embodiments, the method further comprises administering an additional therapeutic agent. In certain embodiments, the additional therapeutic agent is at least one selected from the group consisting of: anti-tumor, antiviral, antibacterial, anti-mycobacterial, anti-fungal, anti-proliferative and anti-apoptotic. In certain embodiments, the additional therapeutic agent is selected from the group consisting of: a growth factor, an anti-inflammatory agent, a vasopressor agent, a collagenase inhibitor, a steroid, a matrix metalloproteinase inhibitor, an ascorbate, an angiotensin, a calreticulin, a tetracycline, a fibronectin, a collagen, a thrombospondin, a transforming growth factors (TGF), a keratinocyte growth factor (KGF), a fibroblast growth factor (FGF), an insulin-like growth factors (IGFs), an IGF binding protein (IGFBP), an epidermal growth factor (EGF), a platelet derived growth factor (PDGF), a neu differentiation factor (NDF), a hepatocyte growth factor (HGF), a vascular endothelial growth factor (VEGF), a heparin-binding EGF (HBEGF), a thrombospondin, a von Willebrand Factor-C, a heparin, a heparin sulfate, and a hyaluronic acid. In another aspect, described herein, is a kit for inhibiting an activity of an inflammasome in an inflammation-affected cell in a subject, the kit comprising: (a) a pharmaceutical composition comprising: (i) a membrane-independent CD59 protein and/or a nucleotide sequence encoding the CD59 protein; or (ii) a soluble CD59 protein, wherein the composition is in a dosage sufficient to inhibit the inflammasome in the inflammation-affected cell in the subject; (b) instructions for use; and (c) a container.

Another aspect described herein, is a method for treating at least one condition selected from: Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis, the method comprising: (a) administering to the subject a composition comprising (i) a nucleotide sequence encoding a membrane independent CD59 protein operably linked to a promoter for expression and secretion of the membrane independent CD59 protein from cells; or (ii) a soluble CD59 protein; the composition inhibiting inflammasome activation. In another aspect, described herein, is a method for inhibiting inflammasome activation in cells of an inflammation-affected subject, the method comprising: (a) measuring in the subject an inflammasome activity marker; and (b) if the inflammasome activity marker is positive administering to the subject a composition comprising: (i) a nucleotide sequence encoding a membrane independent CD59 protein operably linked to a promoter for expression and secretion of the membrane independent CD59 protein in the cells of the inflammation-affected subject; or (ii) a soluble CD59 protein; the composition inhibiting inflammasome activation. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the method further comprises, measuring in the subject an inflammasome activity marker after the administering of the composition comprising the nucleotide sequence encoding the membrane independent CD59 protein operably linked to the promoter for the expression and secretion of the membrane independent CD59 protein or the soluble CD59 protein.

Another aspect described herein, is a method for inhibiting inflammasome activation in cells of an inflammation-affected subject, the method comprising administering to the subject a composition comprising: (a) a nucleotide sequence encoding a membrane independent CD59 protein operably linked to a promoter for expression and secretion of the membrane independent CD59 protein in the cells of the inflammation-affected subject, wherein the composition inhibits inflammasome activation; or (b) a soluble CD59 protein; wherein the subject has displayed positive results for expression or activity of at least one inflammasome activity marker in the inflammation-affected subject. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3). In certain embodiments, the method further comprises, measuring in the subject an inflammasome activity marker after the administering of the composition comprising: (a) the nucleotide sequence encoding the membrane independent CD59 protein operably linked to the promoter for the expression and secretion of the membrane independent CD59 protein; or (b) the soluble CD59 protein. In certain embodiments, the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis. In certain embodiments, the subject has been diagnosed with or is suspected of being afflicted with a uveitis. In certain embodiments, the positive results for expression or activity of at least one inflammasome activity marker in the inflammation-affected subject is elevated expression or activity of at least one inflammasome activity marker in comparison to expression or activity of at least one inflammasome activity marker in a subject not diagnosed or afflicted with Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis. In certain embodiments, the positive results for expression or activity of at least one inflammasome activity marker in the inflammation-affected subject is determined by a histological score.

### Brief Description of Drawings

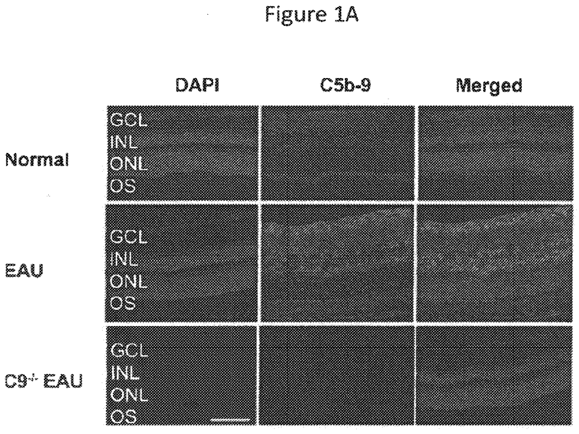

**Figure 1A****-** **Figure 1F** are sets of microphotographs and bar graphs which demonstrate that MAC mediated NLRP3 inflammasome activation increases IL-1β production in EAU. Values are represented as mean ± SEM. GCL, Ganglion cell layer; INL, Inner nuclear layer; ONL, Outer nuclear layer; OS, Outer segments, Scale bar-100µm.
**Figure 1A** is a set of microphotographs of retinal cryostat sections stained with 4',6-diamidino-2-phenylindole (DAPI) (left column) C5b9 antibody (middle column) indicating elevated MAC formation in EAU retina compared to normal control retina in mice. C9^{-/-} EAU mouse retina (bottom row) was observed to be negative C5b9 protein and thus for MAC.

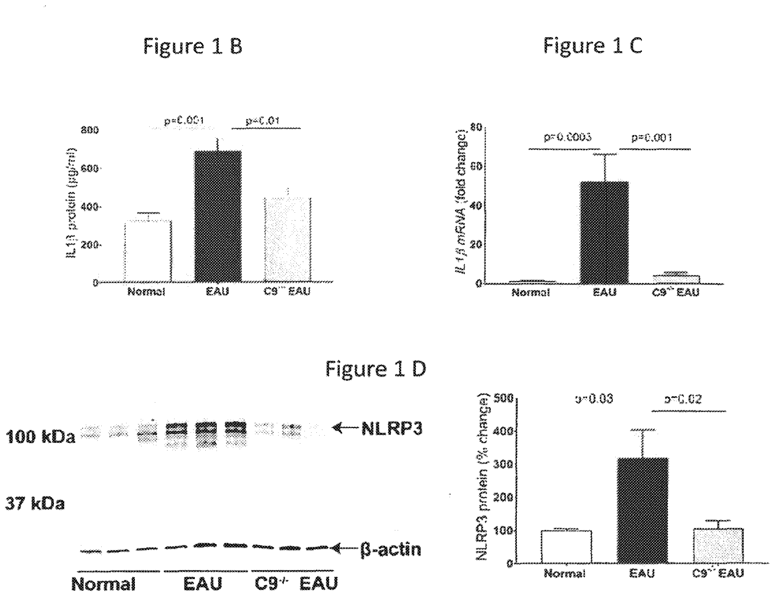

**Figure 1B** is a bar graph of production of IL-1β protein measured by ELISA in EAU retinas (middle bar) and control tissues. The production of IL-1β protein in EAU retinas was observed to be 112% of control retinas, by ELISA, and 45 fold increased by RT-PCR. A 37 % greater IL-1β protein level in C9-/- EAU retinas, and 3.8 fold increase in IL-1β protein and MRNA, respectively, was observed compared to normal control retinas.
**Figure 1C** is a bar graph of production of IL-1β mRNA measured by RT-PCR in EAU retinas (middle bar) and control tissues. The level of IL-1β mRNA in EAU retinas was observed to be 4464% greater than in normal control retinas. IL-1β mRNA level in C9-/- EAU retinas was observed be 285% greater than normal control retinas.
**Figure 1D** is a photograph of a western blot and a bar graph showing that more than 200% greater NLRP3 protein level was observed in EAU retinas compared to normal control retinas of mice. A slightly greater NLRP3 protein level was observed in C9-/-EAU retinas compared to normal control retinas of mice.

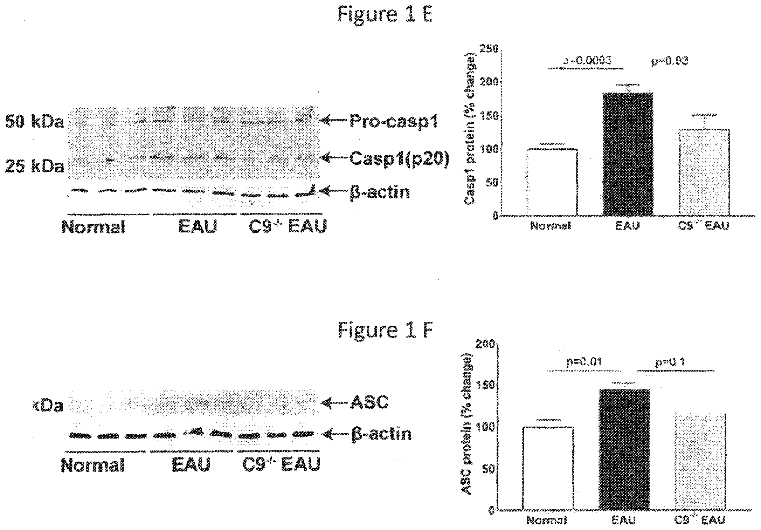

**Figure 1E** is a photograph of a western blot and a bar graph showing that more than 80% greater in Caspase 1(p20) protein levels was observed in EAU retinas compared to normal control retinas of mice. An amount of 30% greater Caspase 1(p20) protein level was observed in C9-/-EAU retinas compared to normal control retinas of mice.
**Figure 1F** is a photograph of a western blot and a bar graph showing 44 % more ASC protein in EAU retinas compared to normal control retinas in mice. The ASC levels in C9-/- EAU mice retinas were observed to be marginally elevated relative to normal control retinas. The western blot and RT-PCR values were normalized to β-actin.

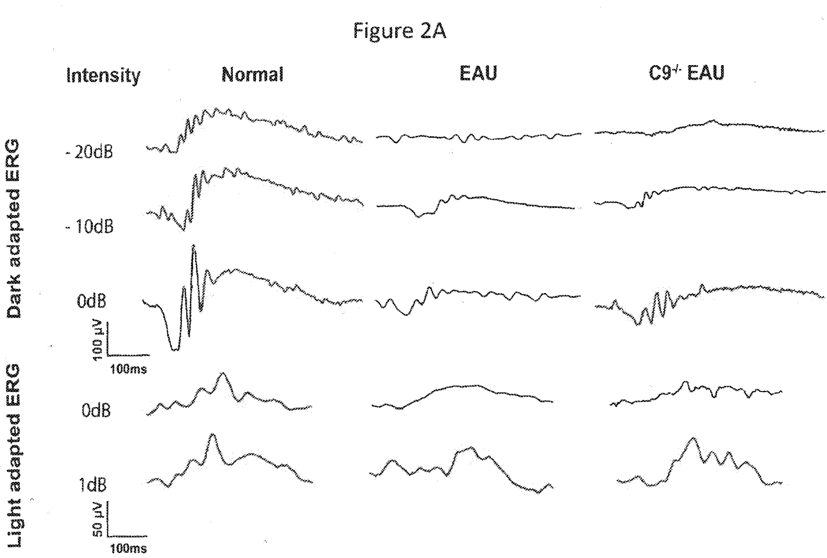

**Figure 2A****-** **Figure 2B** are sets of Electroretinogram (ERG) waveforms and line graphs showing the effects of MAC formation on retinal function in EAU. The data obtained in examples herein indicate that EAU results in extensive damage to photoreceptors, which impairs retinal function in comparison to normal control retinas.
**Figure 2A** is a set of ERG responses in normal, EAU, and C9-/- EAU groups. Dark adapted (scotopic) and light adapted (photopic) responses were analyzed in normal, EAU and C9-/- EAU mice retinas. For dark-adapted ERGs, -20 dB (decibel) (0.025 cds/m²), -10dB (0.25 cds/m²) and 0dB (2.5 cds/m²) flash light intensities were used. For light-adapted ERG, 0dB (2.5 cds/m²) and 1dB (3.15 cds/m²) flash light intensities were used.

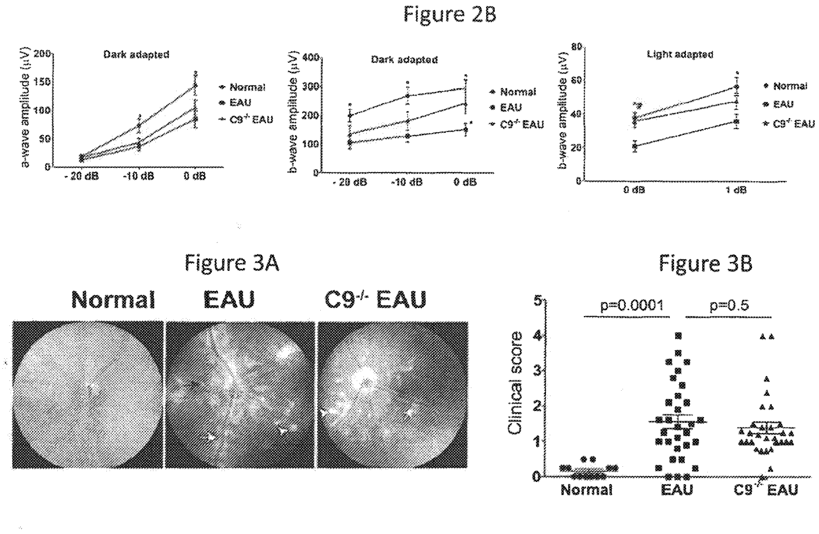

**Figure 2B** is a set of line graphs of dark and light-adapted a-wave ERG amplitudes and b-wave ERG amplitudes plotted with respect to intensities. For dark-adapted ERGs, -20db, -10dB and 0dB flash light intensities were used. For light-adapted ERG, 0dB and 1dB flash light intensities were used. Values are represented as mean ± SEM.*p<0.05, #p<0.05 compared to EAU.
**Figure 3A****-** **Figure 3F** are sets of microphotographs and dot plots showing that C9 ^{-/-} mice are not protected from EAU. In examples herein mice were immunized subcutaneously with 200 µg of IRBP (1-20) peptide in 0.2 mL of Complete Freund's adjuvant (CFA) emulsion containing 2.5 mg/mL*Mycobacterium tuberculosis.* The mice simultaneously were injected with 1.5 µg *Bordetellapertussis* toxin diluted in 100µl Phosphate Buffered Saline (PBS) via an intraperitoneal injection. The funduscopic EAU severity was analyzed after 24 days between normal, EAU and C9-/- EAU mice. Focal lesions, linear lesions, vasculitis, retinal hemorrhages, infiltrates and retinal detachment were examined. According to the severity of these findings, the EAU clinical scores were graded on a scale of 0-4.
**Figure 3A** is a set of microphotographs of Fundus images from EAU and C9-/- EAU groups showing retinal inflammatory infiltrates (white arrow head), vasculitis (white arrow), and retinal folds (black arrow).
**Figure 3B****-****Figure 3F** are dot plots of individual overall clinical scores, vasculature scores, cellular infiltration scores, optic disc scores and structural damage scores respectively. The combination of vasculature score (Figure 3C), cellular infiltration score (Figure 3D), optic disc score (Figure 3E) and structural damage score (Figure 3F) is calculated and is plotted as individual overall clinical score (Figure 3B). Values are represented as mean ± SEM.
**Figure 4A****-** **Figure 4E** is a set of microphotographs and bar graphs of retinal imaging and histology of tissues from EAU, normal control and C9^{-/-} EAU mice.

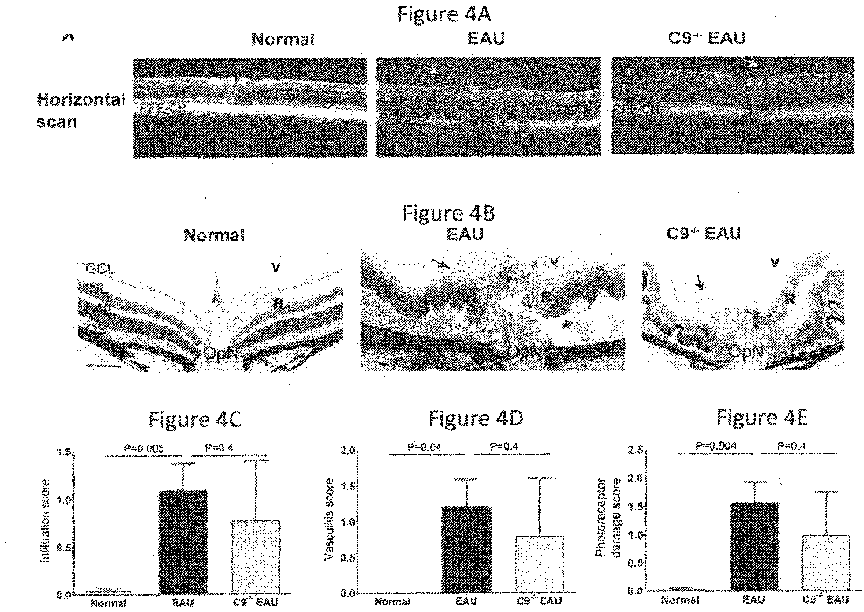

**Figure 4A** is a set of microphotographs of horizontal OCT scans showing folding in the retina (red arrow) and vitreous cellular infiltrates (white arrow) in EAU and C9-/- EAU mice and not in normal control mice.
**Figure 4B** is a set of microphotographs of retinal sections (5µm) which were obtained from paraffin embedded eyes harvested on day 24 post EAU induction and stained with hematoxylin and eosin. Infiltration of inflammatory immune cells in the vitreous (V) are shown by black arrows; black asterisk represents retinal (R) detachment; retinal folds are shown by white arrow heads.
**Figure 4C****-** **Figure 4E** are sets of bar graphs showing the extent of severity of EAU pathology individually scoring cellular infiltration, vasculitis and photoreceptor damage, respectively, as described in examples herein. Values are represented as Mean ± SEM. RPE-CH, RPE and choroid; R, Retina; V, Vitreous; GCL, Ganglion cell layer; INL, Inner nuclear layer; ONL, Outer nuclear layer; OS, Outer segments; OpN, Optic nerve, Scale bar 100µm.

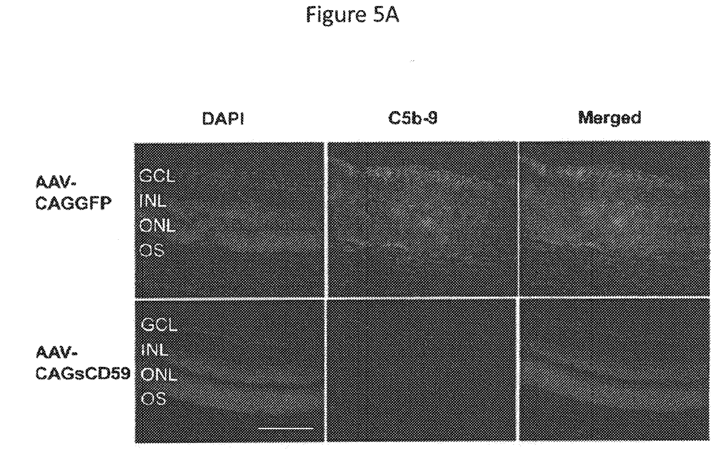

**Figure 5A****-** **Figure 5F** are sets of microphotographs and bar graphs showing that soluble CD59 inhibits NLRP3 inflammasome activation and IL-1β production in EAU.
**Figure 5A** is a set of microphotographs of retinal cryostat sections of eyes from EAU mice injected with either AAVCAGsCD59 or with control AAVCAGGFP, and were stained with C5b9 antibody or with DAPI.

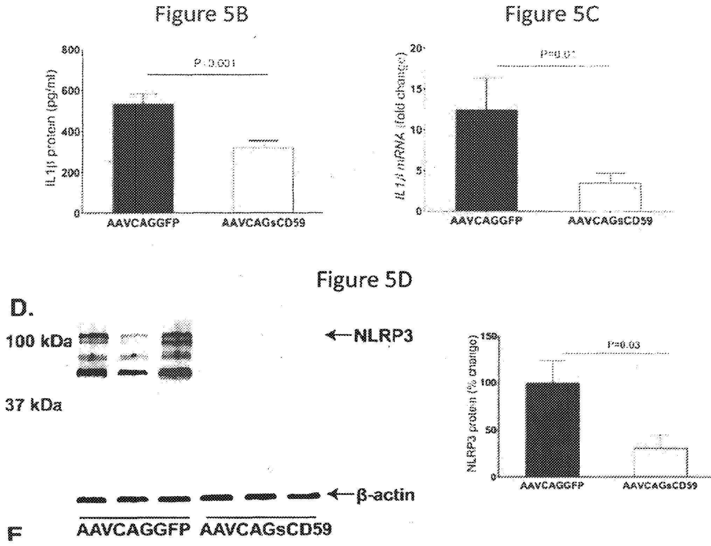

**Figure 5B** is a bar graph of level of IL-1β protein measured by ELISA. The IL-1β protein levels in retinas of EAU mice injected with AAVCAGsCD59 were observed to be 40% lower than in retinas of control EAU mice injected with AAVCAGGFP.
**Figure 5C** is a bar graph of level of IL-1β mRNA measured by RT-PCR. The IL-1β mRNA levels in retinas of EAU mice injected with AAVCAGsCD59 were observed to be 70% lower than in retinas of EAU mice injected with control AAVCAGGFP.
**Figure 5D** is a photograph of a western blot and a bar graph showing 60 % less NLRP3 protein amount in retinas of EAU mice injected with AAVCAGsCD59 compared to retinas of EAU mice injected with control AAVCAGGFP.

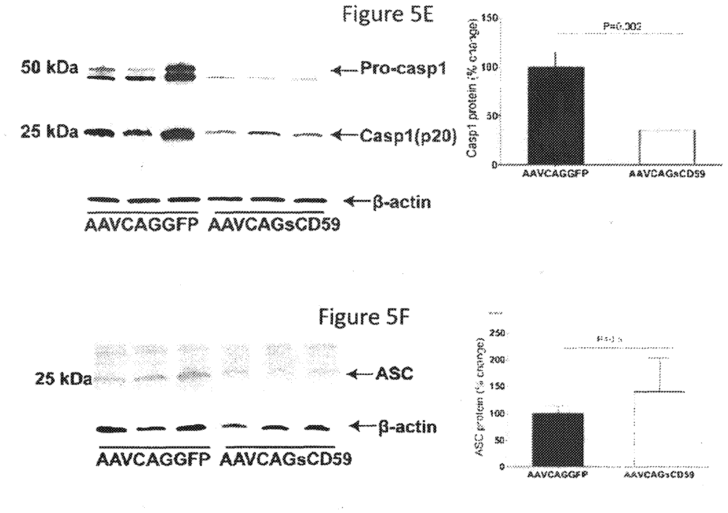

**Figure 5E** is a photograph of a western blot and a bar graph showing 60 % less Caspase 1 (p20) protein in retinas of EAU mice injected with AAVCAGsCD59 than retinas of EAU mice injected with control AAVCAGGFP.
**Figure 5F** is a microphotograph of a western blot and a bar graph showing comparable ASC protein levels in retinas of EAU mice injected with AAVCAGsCD59 and retinas of EAU mice injected with control AAVCAGGFP. The western blot and RT-PCR values are normalized to β-actin. Values are represented as Mean ± SEM. GCL, Ganglion cell layer; INL, Inner nuclear layer; ONL, Outer nuclear layer; OS, Outer segments, Scale bar-100µm.

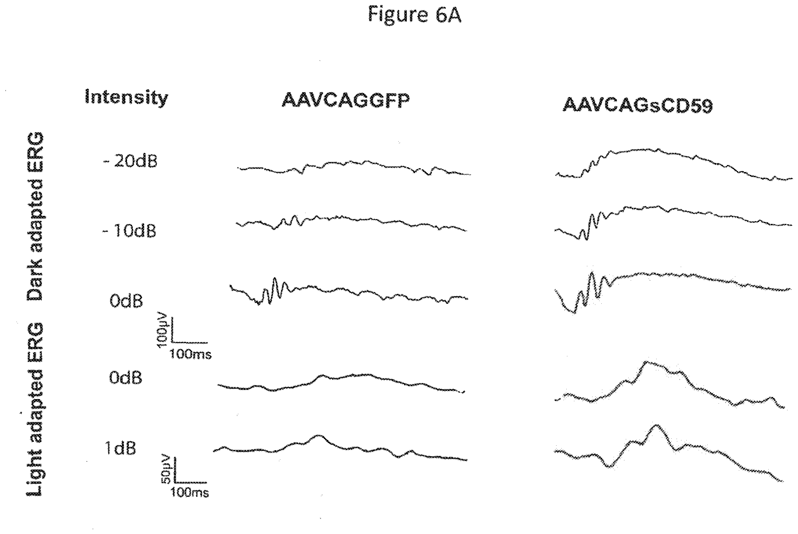

**Figure 6A****-** **Figure 6B** are sets of Electroretinogram (ERG) waveforms and line graphs showing that soluble CD59 improves retinal function in EAU.
**Figure 6A** is a set of ERG responses in EAU mice injected with AAVCAGsCD59 or with control AAVCAGGFP. Both dark adapted (scotopic) and light adapted (photopic) responses were analyzed in retinas of EAU mice injected with AAVCAGsCD59 or with a control AAVCAGGFP. For dark-adapted ERG, - 20dB (0.025 cds/m²), -10dB (0.25 cds/m²) and 0dB (2.5 cds/m²) flash light intensities were used. For light-adapted ERG, 0dB (2.5 cds/m²) and 1dB (3.15 cds/m²) flash light intensities were used.

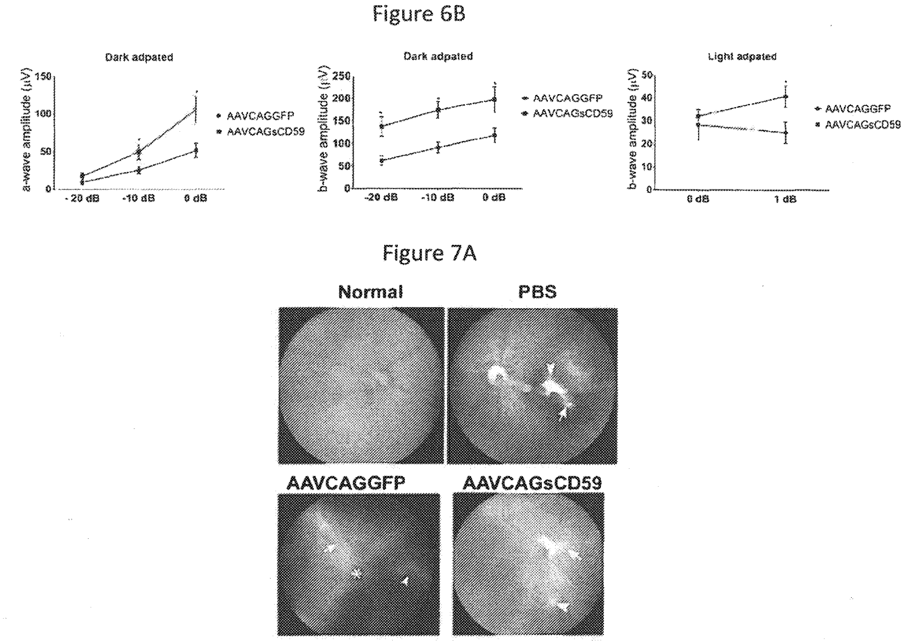

**Figure 6B** is a set of line graphs showing dark and light-adapted a-wave and b-wave ERG amplitudes plotted with respect to intensities. For dark-adapted ERG, - 20db, -10dB and 0dB flash light intensities were used. For light-adapted ERG, 0dB and 1dB flash light intensities were used. ERG responses from C57BL/6J controls are presented in figure 2A. Values are represented as mean ± SEM. *p<0.05, #p<0.05 vs AAVCAGGFP.

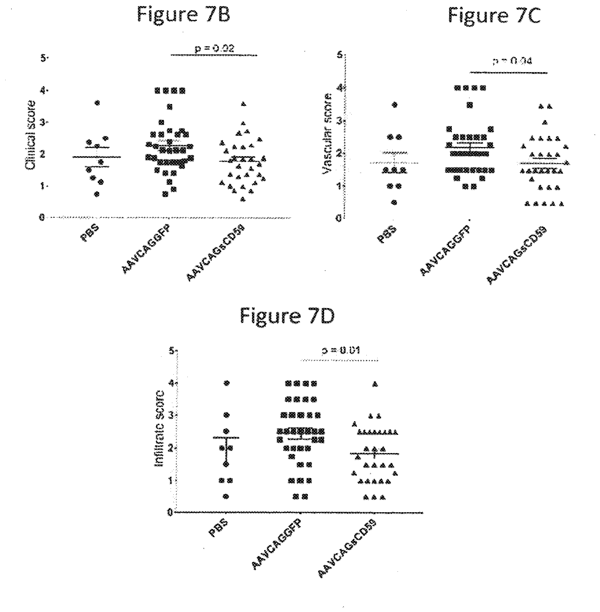

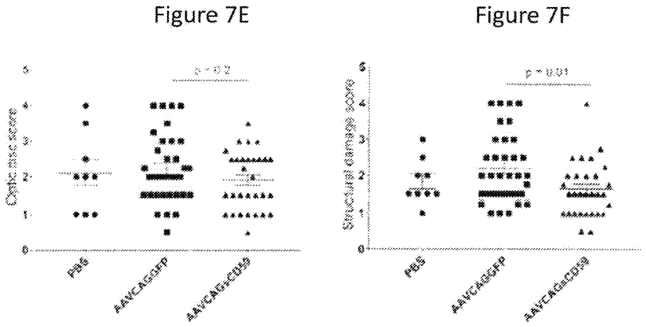

**Figure 7A****-** **Figure 7F** are sets of microphotographs and dot plots showing that soluble CD59 attenuates the severity of clinical score in EAU. The funduscopic EAU severity was analyzed in EAU mice injected with PBS, AAVCAGsCD59 and AAVCAGGFP. The EAU clinical scores were graded on a scale of 0-4.
**Figure 7A** is a set of microphotographs of fundus images from mice injected with PBS, AAVCAGsCD59 or AAVCAGGFP showing retinal inflammatory infiltrates (white arrow head), vasculitis (white arrow), and papilledema (white asterisk).
**Figure 7B****-****Figure 7F** are dot plots of individual overall clinical score, vasculature score, cellular infiltration score, optic disc score and structural damage score, respectively. The combination of vasculature score, cellular infiltration score, optic disc score and structural damage score was calculated and was plotted as individual overall clinical score. Retinas of the group of EAU mice injected with AAVCAGsCD59 were observed to have statistically significant improved individual scores for cellular infiltration, vasculature, and structural damage and overall clinical score, compared to the retinas of the group of mice injected with control AAVCAGGFP. Differences in optic disc scores (Figure 7E) were observed to remain statistically insignificant for EAU mice injected with AAVCAGsCD59 compared to EAU mice injected with control AAVCAGGFP. The differences between PBS and AAVAGGFP injected eyes remained statistically insignificant. Values are represented as mean ± SEM.

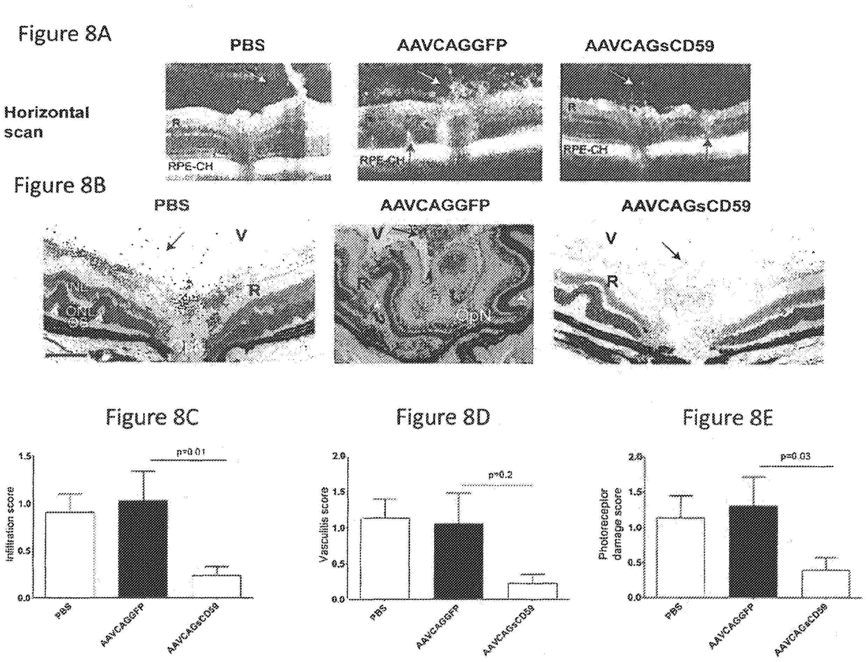

**Figure 8A** **-****Figure 8E** are sets of microphotographs and bar graphs showing effect of CD59 on OCT changes and retinal histology scores in EAU mice.
**Figure 8A** is a set of microphotographs of horizontal OCT scans showing retinal foldings in the retina (red arrow) and vitreous cellular infiltrates (white arrow) in retinas of EAU mice injected with AAVCAGGFP or with control AAVCAGsCD59 or with control PBS.
**Figure 8B** is a set of microphotographs of retinal sections (5µm) which were obtained from paraffin embedded eyes on day 24 post EAU induction and were stained with hematoxylin and eosin. Infiltration of inflammatory immune cells in the vitreous (V) is indicated by black arrows; retinal (R) detachment is indicated by black asterisk; retinal folds are indicated by white arrow heads.
**Figure 8C****-** **Figure 8E** are sets of bar graphs showing extent of severity of EAU pathology individually scored cellular infiltration, vasculitis and photoreceptor damage, respectively, as described in examples herein. Values are represented as mean ± SEM. RPE-CH, RPE and choroid; R, Retina; V, Vitreous; GCL, Ganglion cell layer; INL, Inner nuclear layer; ONL, Outer nuclear layer; OS, Outer segments; OpN, Optic nerve; Scale bar 100µm.

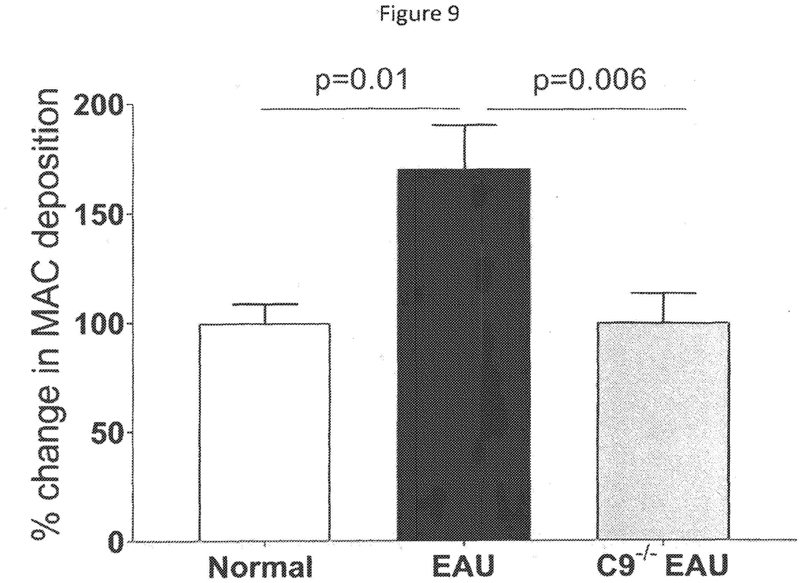

**Figure 9** is a bar graph showing elevation of MAC in EAU retinas. Quantity of MAC fluorescence intensity in retinas of EAU mice was observed to be 70% greater in MAC formation compared to normal control retinas in mice. Values are represented as mean ± SEM.

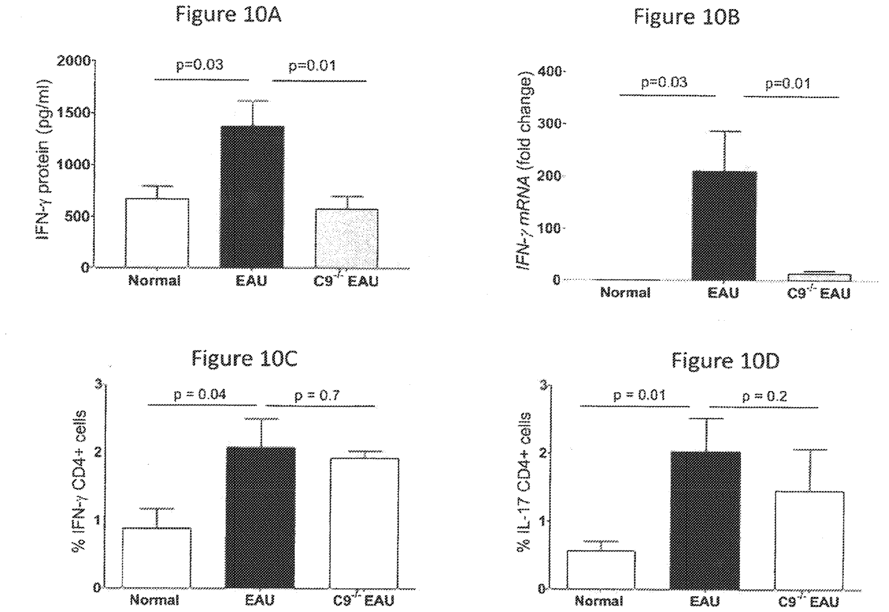

**Figure 10A** **-****Figure 10D** are sets of bar graphs showing effects of MAC on the differentiation of Th1 and Th17 cells in EAU retinas. Freshly dissected retinas were quantified for mRNA and protein levels of IL-17 and IFN-γ using ELISA and RT-PCR in control, EAU and C9^{-/-} EAU retinas.
**Figure 10A** is a bar graph showing that IFN-γ protein level in retinas of EAU mice was 102% and MRNA was 200 fold greater, respectively, than IFN-γ protein expression level in retinas of control normal mice. The IFN-γ protein expression level in C9^{-/-} EAU retinas was observed to be 14% greater, than IFN-γ protein expression level in retinas of control normal mice.
**Figure 10B** is a bar graph showing that IFN-γ mRNA level in retinas of EAU mice was 200 fold greater compared to IFN-γ mRNA level in retinas of control normal mice and protein was 102% of normal. The IFN-γ mRNA level in C9^{-/-} EAU retinas was observed to be 1315%, greater compared than IFN-γ mRNA level in retinas of control normal mice.
**Figure 10C** is a bar graph showing that the IL-17 protein levels in retinas of EAU mice was observed to be 99% greater than IL-17 protein levels in normal control retinas. The IL-17 protein levels in retinas of C9^{-/-} EAU mice were observed to be only 44% greater compared to IL-17 protein levels in retinas of normal control mice.
**Figure 10D** is a bar graph showing IL-17 mRNA levels in retinas of EAU mice and C9-/- EAU mice. The IL-17 mRNA levels in normal control retinas remained below the detection limit. Each experiment was repeated two to three times. Values are represented as mean±SEM.

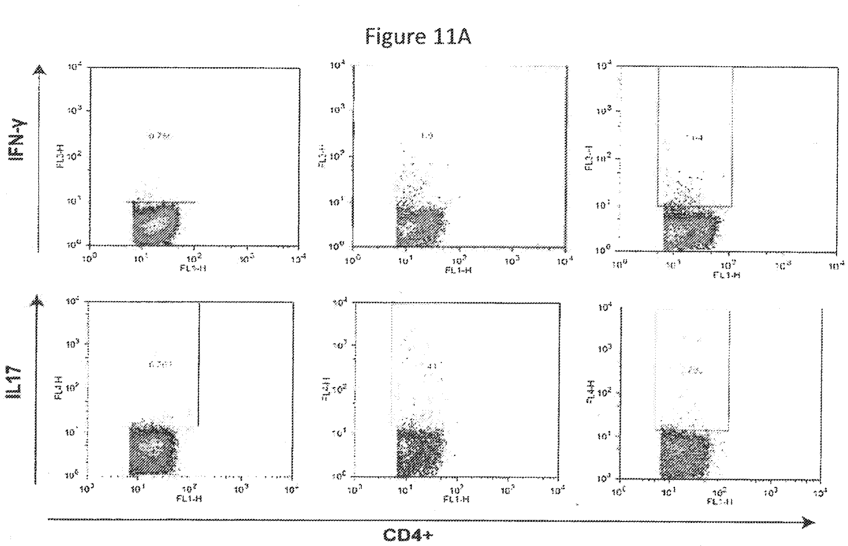

**Figure 11A** **-****Figure 11C** are sets of scatter plots and bar graphs of amounts of IFN-γ or IL-17 in Th1 and Th17 cells in draining lymph node (DLN) in EAU mice. DLN cells were collected from EAU and C9-/- EAU mice 24 days post EAU induction.
**Figure 11A** is a set of scatter plots for cells from the DLN which were stained for IL-17A and IFN-γ plotted with respect to CD4+ cells.

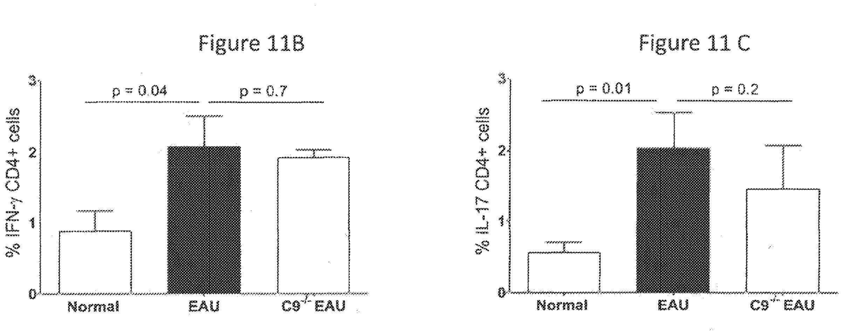

**Figure 11B** and **Figure 11C** are bar graphs showing a significant increase in IL-17 and IFN-γ positive CD4+ cells from draining lymph nodes in EAU mice compared to normal control mice. The difference in percentage of IL-17 positive CD4+ cells and IFN-γ positive CD4+ cells from draining lymph nodes in C9^{-/-} EAU mice relative to EAU mice was observed to be insignificant. Each experiment was repeated three times. Values are represented as mean±SEM.

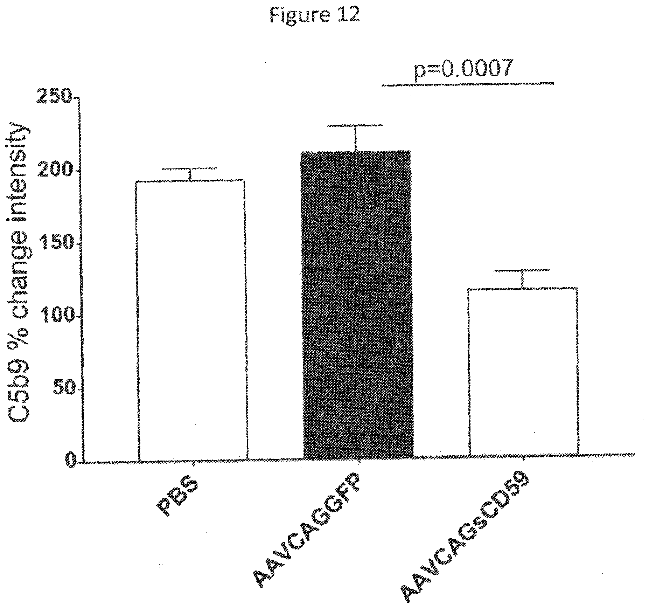

**Figure 12** is a bar graph showing that MAC formation is inhibited by a single intravitreal injection of AAVCAGsCD59 in EAU retinas. Quantity of MAC fluorescence intensity in retinas of EAU mice injected with AAVCAGsCD59 retina was 45% reduced in formation of MAC compared to quantity in retinas of EAU mice injected with control AAVCAGGFP. Values are represented as mean ± SEM.

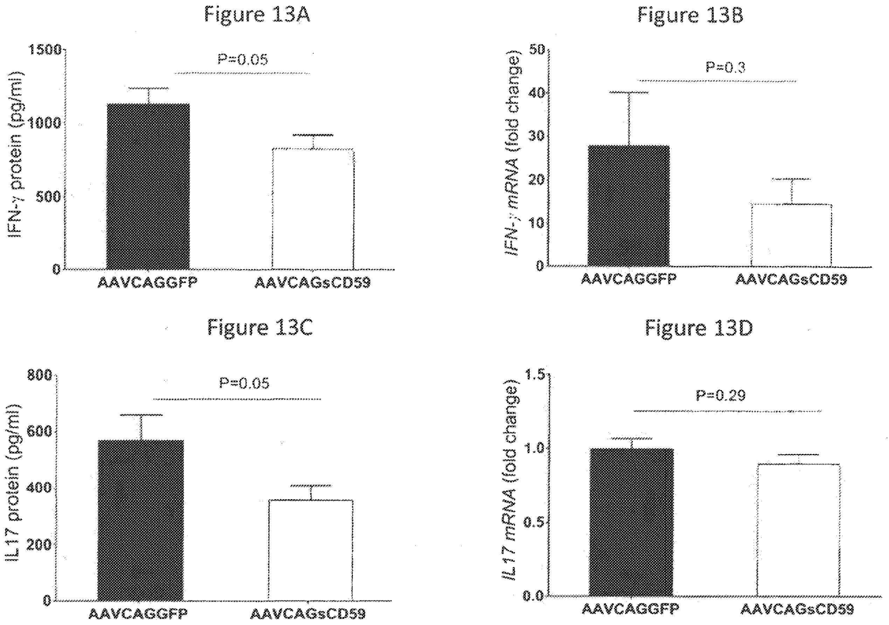

**Figure 13A** **-****Figure 13D** are sets of bar graphs showing that soluble CD59 inhibits differentiation of Th1 and Th17 cells in EAU retinas. Freshly dissected retinas were quantified for mRNA and protein levels of IL-17 and IFN-γ using ELISA and RT-PCR in EAU mice injected with AAVCAGsCD59 and control AAVCAGGFP.
**Figure 13A** is a bar graph showing that IFN-γ protein level was 25% less in retinas of EAU mice injected with AAVCAGsCD59 compared to IFN-γ protein levels in EAU mice injected with control AAVCAGGFP.
**Figure 13B** is a bar graph showing that IFN-γ mRNA expression level was 47% less in retinas of EAU mice injected with AAVCAGsCD59 compared to IFN-γ mRNA levels in EAU mice injected with control AAVCAGGFP.
**Figure 13C** is a bar graph showing that IL-17 protein level was 35% less in retinas of EAU mice injected with AAVCAGsCD59 compared to IL-17 protein levels in EAU mice injected with control AAVCAGGFP.
**Figure 13D** is a bar graph showing that IL-17 mRNA level was 10% less in retinas of EAU mice injected with AAVCAGsCD59 compared to IL-17 mRNA levels in EAU mice injected with control AAVCAGGFP. Differences in amounts of mRNA of IL-17 and IFN-γ were observed to be not statistically significant. Each experiment was repeated two to three times. Values are represented as mean±SEM.

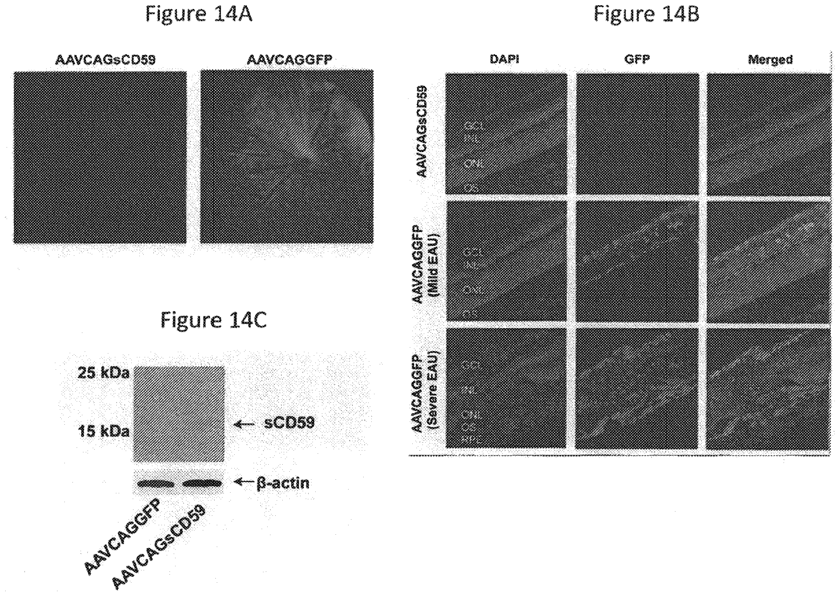

**Figure 14A****-****Figure 14C** are microphotographs and a western blot showing the retinas of six-week-old C57B1/6J mice injected with 3.5 x 10⁹ genome copies/µl of AAVCAGsCD59 or AAVCAGGFP (1 µl) and one week later challenged with EAU and maintained for 24 days.
**Figure 14A** is a Fundus image exhibiting expression of GFP in mouse retina.
**Figure 14B** are retinal cryostat images from the AAVCAGFP injected group exhibiting robust expression of GFP in the ganglion cell layer (top row), inner plexiform layer and inner nuclear layer in retina from mild EAU mouse (middle row), and retina from severe EAU mouse exhibiting GFP expression in photoreceptors and retinal pigment epithelium (bottom row). Top row: control AAVCAGsCD59
**Figure 14C** is a western blot showing expression of sCD59 from mouse retina after a single intravitreal injection of AAVCAGsCD59. Abbreviations: GCL, Ganglion cell layer; INL, Inner nuclear layer; ONL, Outer nuclear layer; OS, Outer segments; RPE, Retinal pigment epithelium.

### Detailed Description

Described herein are methods, compositions, and kits for treating individuals afflicted with inflammasome related disorders with a membrane independent (soluble) CD59 protein. The membrane independent CD59 protein can be expressed from an administered nucleic acid or from direct administration of the protein. Contemplated are nucleic acids encoding a membrane independent CD59 protein or a composition thereof for use in treating an inflammasome related disorder. Also contemplated is a membrane independent CD59 protein or composition thereof for use in treating an inflammasome related disorder. In certain embodiments, the inflammasome related disorder is in the eye of a subject. In certain embodiments, the inflammasome related disorder comprises a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis. In certain embodiments, the inflammasome related disorder comprises a uveitis. The proteins or nucleic acids encoding the soluble/membrane independent CD59 can be administered to a subject that has displayed positive results for expression or activity of at least one inflammasome activity marker. In certain embodiments the positive results are in an eye of the subject. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3).

Immunization of mice with a peptide derived from retinal interphotoreceptor-binding protein (IRBP) results in T-cell mediated autoimmune disease analogous to the clinical and histological features observed in human uveitis (Caspi, R. R., et al. (1988) J. Immunol 140, 1490-1495; Chan, C. C., et al. (1990) J. Autoimmun. 3, 247-255). This experimental model of autoimmune uveitis (EAU) is used as an approach for the study of uveitis and for the development of therapies for this disease, which is used in examples herein.

In order to further understand the potential mechanism by which MAC may be involved in the pathogenesis of EAU and to elucidate the importance of NLRP3 activation and production of IL-1β in the development of EAU, MAC deposition was examined in EAU retina and that MAC may be necessary for the activation of the NLRP3 inflammasome and subsequent release of IL-1β.

MAC is contains one molecule each of C5b, C6, C7, C8 and up to twelve C9 molecules. Since C9-/- mice cannot assemble a functioning MAC, such mice may be partially protected from EAU. In examples herein EAU was induced in control C57BL/6J mice and C9-/- mice and the EAU pathology in the control mice was compared to the EAU pathology in C9-/- mice. The therapeutic potential of inhibiting C9 in EAU was examined by gene therapy approach using an adeno-associated virus (AAV) expressing a soluble CD59 (AAVCAGsCD59), which is a protein that prevents the incorporation of C9 into the preformed C5b-8 complex. The development of EAU in AAVCAGsCD59-injected mice was monitored by fundus imaging, spectral domain optimal coherence tomography (SD-OCT), retinal histopathology and immunohistochemistry. Retinal function was quantified by the electroretinogram (ERG) and NLRP3 inflammasome activation was measured by western blots, enzyme-Linked Immunosorbent Assay (ELISA) and real-time polymerase chain reaction (RT-PCR). The results observed in the examples herein associate for the first time a direct role for MAC as an activator of the NLRP3 inflammasome and IL-1β production in the development of EAU. The data in examples herein demonstrate for the first time that AAV-mediated expression of soluble CD59 is a potential gene therapy for the treatment of uveitis.

In the examples herein, the direct role of MAC in the activation of the NLRP3 inflammasome in EAU was investigated. The data from examples herein demonstrate that MAC is deposited in the EAU retina, which results in activation of the NLRP3 inflammasome and increased production of IL-1β. The examples further demonstrate that C9 ^{-/-} EAU mice fail to form MAC on their retina and concomitantly have attenuated activation of the NLRP3 inflammasome, which indicates a link between deposition of MAC and activation of the NLRP3 inflammasome in EAU. Even though C9-/- mice fail to rescue the EAU pathological phenotype, AAV-mediated delivery of sCD59, an inhibitor of C9 incorporation into MAC, unexpectedly attenuates many aspects of the EAU pathological phenotype including activation of the NLRP3 inflammasome.

The complement system is a major component of innate immunity and consists of a diverse group of plasma and membrane-bound proteins. These membrane-bound proteins play a central role in protection against pathogens and in the regulation of immune and inflammatory processes. The activation of complement against pathogens for host defense is necessary, but over-activated complement may inflict damage to host tissues (Morgan, B. P., et al. (2015) Nat. Rev. Drug Discov. 14, 857-877; Carroll, M. V., et al. (2011) Adv. Drug Deliv. Rev. 63, 965-975). Therefore, it is necessary to maintain a balance between complement activation and complement inhibition by complement regulatory proteins. Over-activated and unregulated complement system terminates in the formation of MAC that has been shown to be involved in a variety of ocular disorders including EAU (An, F., et al. (2009) Invest. Ophthalmol. Vis. Sci. 50, 3778-3782; Copland, D. A., et al. (2010) Clin. Exp. Immunol. 159, 303-314; Read, R. W., et al. (2006) Exp. Eye Res. 82, 389-394; Gehrs, K. M., et al. (2010) Arch. Ophthalmol. 128, 349-358; Yanai, R., et al. (2012) Adv. Exp. Med. Biol. 946, 161-183). However, the direct role of MAC deposition in the pathogenesis of EAU remained to be investigated. In examples herein, it was observed that MAC was deposited in EAU, which led to increased production of IL-1β. However, C9-/- EAU mice that lack the ability to form MAC and consequently were observed to have reduced IL-1β levels.

Although MAC deposition on cell membranes eventually results in cell death by lysis, sublytic MAC on cell membranes has been implicated in regulation of cell cycle and proliferation, apoptosis, production of cytokines and initiation of downstream signaling cascades (Morgan, B. P., et al. (2015) Nat. Rev. Drug Discov. 14, 857-877). The NLRP3 inflammasome complex is a group of cytoplasmic proteins consisting of a primary regulatory subunit NLRP3, an adapter subunit ASC and the effector subunit caspase-1 which converts pro-IL-1β into active IL-1β (Latz, E., et al. (2013) Nat. Rev. Immunol. 13, 397-411; Broz, P., et al. (2016) Nat. Rev. Immunol. 16, 407-420). Recent studies in LPS-primed models have shown that sublytic MAC-induced pore formation which led to an accumulation of intracellular Ca2+ that subsequently activated the NLRP3 inflammasome (Triantafilou, K., et al. (2013) J. Cell Sci. 126, 2903-2913; Laudisi, F., et al. (2013) J. Immunol. 191, 1006-1010). The NLRP3 inflammasome has been previously implicated in several ocular diseases (Devi, T. S., et al. (2012) Exp. Diab. Res. 2012, 438238; Tseng, W. A., et al. (2013) Invest Ophthalmol Vis Sci 54, 110-120). The observations in examples herein demonstrated that attenuation of the activation of NLRP3 inflammasome is a novel therapeutic approach for the treatment of uveitis. The examples herein demonstrate for the first time the direct role of MAC and NLRP3 inflammasome activation in the EAU mouse model. The data obtained in the examples herein demonstrate that MAC directly activated and increased protein expression of NLRP3, Caspase-1 and ASC subunits. These activated subunits form the NLRP3 inflammasome complex which produces active IL-1β. C9-/- EAU mice did not activate the NLRP3 inflammasome and IL-1β remained close to basal levels, confirming activation of the NLRP3 inflammasome as a MAC-dependent pathway.

Autoreactive effector CD4+ T cells are associated with pathogenesis in EAU. Both Th1 and Th17 lineages are specifically responsible in the development of EAU and have been reported in uveitis patients (Amadi-Obi, A., et al. (2007) Nat. Med. 13, 711-718; Caspi, R. R., et al. (1996) J. Immunol. 157, 2668-2675). Further, IL-1β signaling promotes differentiation of CD4+ T cells into Th17 cells (Chung, Y., et al. (2009) Immunity 30, 576-587). Recent studies have shown that blocking the IL-1 signaling pathway can potentially treat EAU in mice (Wan, C. K., et al. (2016) J. Immunol. 196, 543-546). The IL-1R antagonist *anakinra,* soluble decoy IL-1R *rilonacept,* and IL-1b-neutralizing antibody *canakinumab* have been approved for the treatment of uveitis (Knickelbein, J. E., et al. (2017) Handb. Exp. Pharmacol. 242, 231-268). However, multiple side effects and short duration of action makes them limited in use. In the examples herein, increased production of IL-1β was observed in mice retinas affected with EAU; however, lower IL-1β levels were observed in C9-/- mouse retinas affected with EAU. Further, increased differentiation of Th1 and Th17 cells was observed in the mice retinas affected with EAU; however, decreased Th1 and Th17 cells was observed in the C9-/- retinas affected with EAU as indicated by the respective IL17 and IFN-γ protein and mRNA levels. Further, increased levels of Th1 and Th17 positive CD4 cells were observed in DLN from EAU mice. However, levels of Th1 and Th17 positive CD4 cells were observed to remain unchanged in DLN from C9-/- EAU mice. Without being limited by any particular theory or mechanism of action, it is here envisioned that MAC-induced IL-1β production and differentiation of Th1 and Th17 cells in EAU retina is a local effect.

Many complement regulatory proteins are secreted or found on the surface of cells to keep complement mediated damage to host tissues in check. These proteins include Factor H, decay accelerating factor (CD55), membrane cofactor protein (CD46) and protectin (CD59). The decreased activity or deficiency of these complement regulatory proteins can lead to immunopathologies including EAU and experimental autoimmune anterior uveitis (Morgan, B. P., et al. (2015) Nat. Rev. Drug Discov. 14, 857-877; An, F., et al. (2009) Invest. Ophthalmol. Vis. Sci. 50, 3778-3782; Carroll, M. V., et al. (2011) Adv. Drug Deliv. Rev. 63, 965-975; Jha, P., et al. (2006) J. Immunol. 176, 7221-7231). Autoimmune uveitis is a chronic and multifactorial disease associated with systemic disease. Various studies have shown that inhibition of complement activation may help in ameliorating EAU pathology in mice (An, F., et al. (2009) Invest. Ophthalmol. Vis. Sci. 50, 3778-3782; Copland, D. A., et al. (2010) Clin. Exp. Immunol. 159, 303-314; Read, R. W., et al. (2006) Exp. Eye Res. 82, 389-394). Embodiments of the methods herein demonstrate a long-acting gene therapy approach utilizing AAV to deliver sCD59 to EAU mice. For the first time AAVCAGsCD59 is demonstrated to inhibit MAC deposition in EAU retina. The examples herein demonstrate that AAVCAGsCD59 inhibit the activation of the NLRP3 inflammasome and attenuated IL-1β production.

Further, the examples herein demonstrate that a single intravitreal injection of AAVCAGsCD59 inhibited phenotypic pathologies in EAU mice. In these mice, the clinical signs associated with EAU were significantly improved, such as reduced inflammation, fewer immune cell infiltrates and reduced vasculitis. The data obtained in the examples herein demonstrate that AAVCAGsCD59 injection leads to an improvement in loss of retinal function associated with EAU in both dark and lighted adapted ERGs. In the examples herein C9 -/- mice were observed to significantly inhibit activation of the NLRP3 inflammasome and observed to reduce upregulation of IL-1β. Although a trend of improved histology score and improved retinal function was observed in C9-/- EAU mice the differences were observed to not reach a significant level of statistical significance except for a few data points in ERG.

Unexpectedly, our studies do suggest that AAVCAGsCD59 possibly attenuates inflammation by a means other than blocking incorporation of C9 into the C5b-8 complex. GPI-anchored CD59 has been previously reported to bind to CD2 and transduce activation signals within T cells (Deckert, M. et al. (1995) Eur J Immunol. 25, 1815-22). CD59 cross-linking induces T cell receptor zeta/ZAP-70 signaling cascade and interleukin-2 (IL-2) synthesis. IL-2 has been found to be successful in modulating the immune system in diseases including type 1 diabetes and vasculitis (Hartemann, A. et al. (2013) The Lancet. Diabetes & Endocrinology. 1, 295-305), supporting the hypothesis that sCD59 may attenuate inflammation in a MAC independent manner. The EAU model used herein for analyzing uveitis has inconsistencies related to mild to moderate EAU development. These minor changes may significantly affect the outcome.

Uveitis is a chronic inflammatory disease and many patients face episodes of relapse and some of them are refractory to available therapies. Overall, conventional therapies remain limited in managing severe and advanced stages of uveitis in patients with systemic autoimmune disease (Bechet's disease, Vogt-Koyanagi disease, etc.) due to significant side effects and short-term efficacy. The treatment for managing uveitis has not advanced significantly during the previous few decades (Knickelbein, J. E., et al. (2017) Handb. Exp. Pharmacol. 242, 231-268). Considering these concerns, it is practical to develop a long-acting therapy such as the continuous inhibition of MAC dependent activation of the inflammasome in uveitis patients. Therefore, single intravitreal injection of AAVsCD59 has significant potential and advantages relative to the short-term effect of present therapies. It has been shown that AAV dependent gene therapies are successful in treating ocular diseases in animal models (Adhi, M., et al. (2013) PLoS One 8, e79661; Ildefonso, C. J., et al. (2015) Hum. Gene Ther. 26, 59-68). Further, AAV-mediated gene therapies have successfully advanced into human clinical trials for age-related macular degeneration (Mingozzi, F., et al. (2011) Nat. Rev. Genet. 12, 341-355).

The examples herein demonstrated that MAC is an important regulator of NLRP3 inflammasome activation and production of IL-1β in EAU. MAC plays an important role in differentiation of Th1 and Th17 cells by increasing IL-1β production. AAV-mediated expression of sCD59 was observed to efficiently inhibit MAC deposition and subsequently inhibit NLRP3 inflammasome activation. A single intravitreal injection of AAVCAGsCD59 was observed to efficiently inhibit the development of EAU in mice.

### CD59 protein

CD59 is a membrane-bound glycoprotein found associated with membranes of cells including human erythrocytes, lymphocytes, and vascular endothelial cells. CD59 protein inhibits assembly of functional MACs and thus protects cells from complement-mediated activation and/or lysis.

Without being limited by any particular theory or mechanism of action, it is here envisioned that plasma membranes of cells are normally protected from the effects of complement by cell-surface proteins, e.g., CD59, that specifically inhibit activation of the C5b-9 pore upon C9 complement protein binding to membrane C5b-8 (Holguin et al. 1989 J. Clin. Invest. 84: 7-17; Sims et al. 1989 J. Biol. Chem. 264: 19228-19235; Davies et al. 1989 J. Exp. Med. 170: 637-654; Rollins et al. 1990 J. Immunol. 144: 3478-3483; and Hamilton et al. 1990 Blood 76: 2572-2577). CD59 competes with C9 complement protein for binding to C8 complement protein in the C5b-8 complex, thereby decreasing or preventing the formation of the C5b-9 membrane attack complex. CD59 thus acts to reduce both cell activation and cell lysis by terminal complement MACs.

Mature human CD59 protein is composed of 77 amino acids and has a molecular weight of 18-21 kD. Precursor human CD59 protein includes an amino-terminal signal peptide of 25 amino acids and a carboxyl-terminal peptide of 26 amino acids which results in attachment of a membrane anchor. Amino acid sequences of examples of precursor human CD59, a mature human CD59, and CD59 sequences of other mammals, e.g., baboon, African green monkey, owl monkey, marmoset, HVS-15, pig, rabbit, rat, and mouse, are shown in Sims et al. U.S. patent number 7,166,568 issued January 23, 2007 (incorporated by reference).

Protein structure of CD59 includes a single cysteine-rich domain, a hydrophobic core with three loops and a small fourth helical loop (Yu et al. 1997 Journal of Experimental Medicine 185(4): 745-753).

The structure and sequence of the gene encoding CD59 has been characterized (Fodor et al. U.S. patent number 5,624,837 issued April 29, 1997; incorporated by reference). The gene is located on the short arm of chromosome 11 in humans, specifically chromosome 1 1p13 and 1 1p14 (Online Mendelian Inheritance in Man accession number and107271), and consists of 4 exons spanning 20 kb (Petranka et al. 1992 Proc. Nat. Acad. Sci. 89: 7876-7879). An untranslated first exon is preceded by a G and C-rich promoter region that lacks a consensus TATA or CAAT motif. The second exon encodes the hydrophobic leader sequence of the protein, and the third exon encodes the N-terminal portion of the mature protein. The fourth exon encodes the remainder of the mature protein, including the hydrophobic sequence for glycophosphoinosital (GPI) anchor attachment to a cell membrane.

CD59 is a glycosylphosphatidylinositol-anchored glycoprotein that is expressed on human peripheral blood leukocytes, erythrocytes, and many cell lines. The protein is expressed on both hematopoietic and non-hematopoietic cells, for example on endothelial cells, peripheral nerve fibers, neurons, microglia, oligodendrocytes, astrocytes, ependymal cells, epithelial cells, acinar cells of the salivary glands, bronchial epithelium, renal tubules and squamous epithelium. See Nose, M. et al. 1990 Immunology 70(2): 145-149; Vedeler, C. et al. 1994 Immunology 82(4): 542-547; and Hidestima, T. et al. 1990 Immunology 69(3): 396:401, each of which is incorporated herein by reference in its entirety. A cDNA encoding CD59 was reported by Sawada, R. et al. 1989 Nucleic Acids Res 17(16): 6728. CDNA encoding CD59 has also been cloned from human T-cell leukemia (YT) and human erythroleukemia (K562) cell lines, and CD59 has been transiently expressed in COS cells (Walsh, L.A. et al. 1990 Eur J. Immol 21(3): 847-850). Human CD59 includes 26 amino acids located at the C terminus, which specifies a signal sequence for attachment of a glycosyl phosphatidyl inositol anchor (GPI anchor) at amino acid asparagine at position 77. A cDNA sequence of CD59 is shown in Fodor et al., U.S. patent number 5,624,837 issued April 29, 1997, which is incorporated herein by reference in its entirety.

Analysis of the physical association of CD59 with components of MAC shows that separate binding sites for CD59 are contained within the α-chains of each of human C8 and human C9. The binding site for interactions of human CD59 with human C9 has been identified as amino acid residues 42 to 58 in the sequence of mature human CD59, that bind to the region of human C9 corresponding to human amino acid residues 334 to 418 of that protein, more particularly human C9 amino acid residues 359 to 384, immediately C-terminal to the predicted membrane-inserting domain of C9 (Sims et al. PCT/US96/17940 filed November 8, 1996 which is incorporated herein by reference in its entirety).

The active surface exposed amino acid residue side chains that are available to bind C8/C9, identified from solution structure of mature human CD59 from published NMR data and the knowledge of the active portion of the CD59 molecule, are histidine at position 44, asparagine at position 48, aspartic acid at position 49, threonine at positions 51 and 52, arginine at position 55, and glutamic acid at position 58. NMR structures for CD59 are described in deposits by Kieffer et al., Human Complement Regulatory Protein CD59 (Extracellular Region, Residues 1 70; NMR, 10 Structures), MMDB Id: 891, PDB Id: 1ERH; Kieffer et al., Human Complement Regulatory Protein CD59 (Extracellular Region, Residues 1 70; NMR, Restrained), MMDB Id: 890, PDB Id: 1ERG; Fletcher et al., CD59 Complexed With Glcnac-Beta-1,4-(Fuc-Alpha-1,6)-Glcnac-Beta-1 (NMR, 10 Structures), MMDB Id: 498, PDB Id: 1CDS; Fletcher et al., CD59 Complexed With Glcnac-Beta-1,4-Glcnac-Beta-1 (NMR, 10 Structures), MMDB Id: 497, PDB Id: 1CDR. The 1CDS and 1CDR deposits by Fletcher et al. Amino acid sequences of CD59 that present these side chains at the same relative positions function in a manner similar to human CD59 (Sims et al.), and such variants are within the scope of the methods, kits and pharmaceutical compositions herein.

The relationship between complement activation and abnormal levels of autoantibodies has been analyzed with respect to ocular diseases such as macular degeneration and other conditions. Hageman et al. U.S. patent application number 2005/0287601 published December 29, 2005 (incorporated by reference) proposes diagnosing macular degeneration by measuring presence of autoantibodies specific for a retinal protein (RPE and choroid proteins) in samples from AMD patients.

Theories have connected causation of Alzheimer's disease and age-related macular degeneration by complement, and prevention inhibiting activation of the complement system and formation of MAC. Dinu U.S. patent application number 2007/0196367 A1 published August 23, 2007 (incorporated by reference) proposes preventing debris formation by inhibiting complement as a therapeutic for Alzheimer's disease and AMD. Patil et al. U.S. patent application number 2007/0203190 A1 published August 30, 2007 (incorporated by reference) lists hydroxylamine compounds (e.g., TEMPOL-H, TEMPO-H, and OXANO-H) or ester derivatives as putative inhibitors of complement activation.

Tomlinson et al., U.S. patent application number 2005/0265995 published December 1, 2005 (incorporated by reference) inhibits complement-directed proteinuria in rats using an agent produced by linking each of complement inhibitors Crry and CD59 at the amino-terminus to single-chain antibody (scFv) that binds to rat glomerular epithelial cells and proximal tubular epithelial cells. Soluble CD59 is described in this reference as ineffective as an inhibitor. Bora et al. 2007 J. Immunol 178: 1783-1790 uses recombinant methods to produce a membrane targeting composition by fusing the binding arm, Fc, of an immunoglobulin G (IgG1) to CD59 protein (rsCD59a-Fc), and injects this fusion into mice by intravenous, intra-ocular (intravitreal) and intra-peritoneal routes. Numbers of CNV-positive spots were reduced in subjects treated with the fusion protein by the intraperitoneal route compared to intravitreal and intravenous routes. The Fc functionality may result in immediate binding upon contact of the fusion protein generally and non-specifically to cells following administration. Administering purified protein is further limited by metabolism and half-life due to presence of proteases and peptidases. Tomlinson et al. 2009 IOVS 50(7): 3056-3064 reduced the size of CNV spots in a mouse model by intravenously injecting the animals having CNV spots with a plasmid encoding a fusion protein complement inhibitor, produced by linking the N-terminus binding domain of factor H to a fragment of complement receptor 2 (CR2) that targets membrane molecules on cells.

A CD59 composition provided herein lacks the primary amino acid sequence for a functional GPI anchor. A functional equivalent protein includes a modified GPI anchor domain amino acid sequence that is functionally defective and lacks the ability to target a membrane. A sCD59 is an example of a recombinant membrane-independent CD59 (rmiCD59). Additional methods of obtaining membrane-independent CD59 include non-recombinant methods such as providing an inhibitor of membrane association, for example, synthesizing CD59 *in vivo* or *in vitro* such that the GPI anchor is lacking. Methods of obtaining the membrane-independent CD59 are shown in examples herein. Additional recombinant techniques for altering the nucleic acid sequence and amino acid sequence of a molecule are well known in the art of genetics and molecular biology.

In various embodiments, the CD59 protein, described herein, is a soluble CD59 protein. The composition provides a CD59 protein and includes a full length nucleic acid of CD59 that had been modified to remove the signal sequence for attachment of the GPI anchor at the nucleotides encoding amino acid asparagine at position 77. Alternatively the nucleic acid sequence of CD59 was modified by point mutations, substitutions or deletions to obtain a nucleic acid sequence that encodes an amino acid sequence that has a modified amino acid sequence at the GPI anchor location, such that the protein would be unable to attach to a membrane of a cell.

The term "membrane independent" as used herein refers to a CD59 amino acid sequence that lacks a GPI anchor or has a modified GPI anchor that lacks function and ability to bind to a cell membrane or a cell-membrane-associated structure such as a membrane-bound protein. A GPI anchor can be modified through recombinant DNA technology to remove the GPI anchoring domain or to introduce one or more mutations to disrupt GPI function. These one or more mutations can comprise an insertion of one or more amino acids, a deletion of one or more amino acids, or a substitution of one or more amino acids.

GPI anchoring involves a multi-step pathway in the endoplasmic reticulum including the interaction of numerous gene products. Many proteins including CD59 require GPI to be expressed at the cell surface and to function effectively. The mechanism by which structure in a protein signal encodes for attachment of GPI anchors is reviewed by Orlean et al. 2007 JLR 48: 993-1011. GPI attachment involves an amino acid sequence that contains: a hydrophobic N-terminal secretion signal that targets the protein to the ER, and a C-terminal GPI signal anchor sequence. In addition to the native CD59 secretion signal which is located at the amino terminus of the protein and is cleaved *in vivo,* other secretion signals are suitable for CD59 protein and are within the scope of the methods herein. General eukaryotic secretion signals that are suitable for use in mammalian cells here are described for example in Ding et al. U.S. patent number 6,733,997 B1 issued May 11, 2004; Tan et al. 2002 Protein Engineering 15(4): 337-345; and Tan et al. 1999 Biochim. Biophys. Acta 1452: 103-120, each of which is incorporated herein by reference in its entirety.

The amino acid to which the GPI becomes linked is referred to as the omega (ω) residue, with amino acids N-terminal to the omega residue referred to as omega-minus (ω-) and with amino acids C-terminal to the omega residue referred to as omega-plus (ω+). The GPI anchor sequence includes a stretch of about ten polar amino acids (i.e., ω-10 to ω-1), for example arginine, lysine, aspartate, glutamate, asparagine, or glutamate, that form a flexible linker region. The ω residue has been observed to be one of: glycine, alanine, serine, asparagine, aspartic acid, or cysteine. Mutation including substitution and deletion of nucleic acids encoding amino acids at omega positions are used to reduce or eliminate the attachment of the GPI anchor or reduce or eliminate the effective functionality of the GPI anchor. For example, such a variation includes substituting the nucleic acids encoding hydrophobic leucine (e.g., nucleic acids CTG) and alanine (e.g., nucleic acids GCA) with nucleic acids encoding glutamine (e.g., nucleic acids CAG) and glutamic acid (e.g., nucleic acids GAA), or glycine (e.g., nucleic acids GGN)which are less hydrophobic (i.e., more hydrophilic) amino acids. Alternatively, a variation includes substituting the ω residue with another amino acid, for example substituting a glycine for a tyrosine.

Other promoter sequences that are useful to regulate transcription of CD59 gene sequences are within the scope of expression of the vectors herein. These promoters are for example constitutive promoters, cell cycle-specific promoters, ubiquitous promoters, tissue-specific promoters, metabolically regulated promoters, inducible promoters, and promoters that are found in specific subjects including humans and animals. Examples of promoters and promoter systems are shown for example in Evans et al. U.S. patent number 6,677,311 B1 issued January 13, 2004; Clark et al. U.S. patent number 7,109,029 B2 issued September 19, 2006; and Hallenbeck et al. U.S. patent number 5,998,205 issued December 7, 1999, each of which is incorporated herein by reference in its entirety.

In the remainder of the amino acid sequence of the portion of CD59 protein which is not involved in GPI anchoring, the scope of the CD59 protein herein is envisioned to include conservative sequence modifications. As used herein, the term "conservative sequence modifications" refers to amino acid modifications that do not significantly affect or alter the characteristics of the CD59 protein or membrane-independent CD59 containing the amino acid sequence, i.e., amino acid sequences of CD59 that present these side chains at the same relative positions will function in a manner similar to human CD59. Such conservative modifications include amino acid substitutions, additions and deletions. Modification of the amino acid sequence of CD59 is achieved using any known technique in the art *e.g*., site-directed mutagenesis or PCR based mutagenesis. Such techniques are described in Sambrook et al., Molecular Cloning: A Laboratory Manual, Cold Spring Harbor Press, Plainview, NY, 1989, Ausubel et al., Current Protocols in Molecular Biology, John Wiley & Sons, New York, NY, 1989, Molecular Biomethods Handbook, 2nd ed., J. M. Walker et al., Humana Press, 2008, and Handbook of Molec. and Cellul. Methods in Biol. and Med., 3rd ed., L. J. Ceske et al., CRC Press, 2011.

Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

In certain embodiments, the CD59 amino acid sequence is an amino acid sequence that is substantially identical to that of the wild type sequence. The term "substantially identical" is used herein to refer to a first amino acid sequence that contains a sufficient or minimum number of amino acid residues that are identical to aligned amino acid residues in a second amino acid sequence such that the first and second amino acid sequences can have a common structural domain and/or common functional activity. For example, amino acid sequences that contain a common structural domain having at least about 60% identity, or at least 75%, 80%, 85%, 90%, 95%, 96%, 98%, or 99% identity. In certain embodiments, the CD59 amino acid sequence is an amino acid sequence that is "identical" to that of the wild type sequence. An identical sequence is one that exhibits 100% identity to a wild type sequence of CD59. In a certain embodiment, an identical sequence can be flanked on the N or C-terminus by one or more amino acids. For example the one or more amino acids can comprise an epitope tag, a purification tag, the remnant of a cleavage site introduced to allow a purification tag to be removed, or one or more modifications to improve stability or bioavailability of the protein.

As used herein the term "about" refers to a number, measurement, or quantity that is near the stated amount by 10% or less.

CD59 polypeptides are useful for the treatment of inflammasome mediated disorders. The amino acid sequence of a human CD59 polypeptide useful for the methods described herein is set forth by the sequence: MGIQGGSVLFGLLLVLAVFCHSGHSLQCYNCPNPTADCKTAVNCSSDFDACL ITKAGLQVYNKCWKFEHCNFNDVTTRLRENELTYYCCKKDLCNFNEQLENG GTSLSEKTVLLLVTPFLAAAWSLHP (SEQ ID NO: 1). The signal sequence of CD59 is cleaved before secretion from the cell, and is dispensable for therapeutic utility. However, nucleic acids that encode a CD59 with a signal sequence increases secretion of a CD59 polypeptide, and, thus, a signal sequence may be desirable to include in CD59 polypeptides encoded by nucleic acids. A signal sequence cleaved human CD59 polypeptide useful for the methods described herein is set forth by the sequence: LQCYNCPNPTADCKTAVNCSSDFDACLITKAGLQVYNKCWKFEHCNFNDVT TRLRENELTYYCCKKDLCNFNEQLENGGTSLSEKTVLLLVTPFLAAAWSLHP (SEQ ID NO: 2). However, the exact signal sequence end may vary from SEQ ID NO: 2 by 1, 2, 3, 4, 5, 6, 7, 8, 9, 10, 11, 12, 13, 14, 15, 16, 17, 18, 19, 20, 21, 22, 23, or 24 amino acids from the first leucine of SEQ ID NO: 2; in either the N-terminal or C-terminal direction of the full CD59 polypeptide set forth in SEQ ID NO: 1. A further deletion of the N-terminal 26 amino acids deletes the GPI anchoring domain of human CD59. A signal sequence cleaved, soluble human CD59 polypeptide useful for the methods described herein is set forth by the sequence: LQCYNCPNPTADCKTAVNCSSDFDACLITKAGLQVYNKCWKFEHCNFNDVT TRLRENELTYYCCKKDLCNFNEQLEN (SEQ ID NO: 3). This soluble version can also vary at its exact N-terminal end by 1, 2, 3, 4, 5, 6, 7, 8, 9, 10, 11, 12, 13, 14, 15, 16, 17, 18, 19, 20, 21, 22, 23, or 24 amino acids, resulting in further deletion from its N-terminus or restoration of its N-terminus. In certain embodiments, the soluble version of CD59 comprises an amino acid sequence as set forth in SEQ ID NO: 2 with one or more mutations to a residue required for GPI attachment. In certain embodiments, the residues are any one or both of the asparagines at amino acid 70 or 77 of SEQ ID NO: 2. In certain embodiments, the CD59 polypeptide useful for the methods described herein comprises deletion of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acids from the N-terminal end, C-terminal end, or both ends of any one of SEQ ID NOs: 1, 2, or 3. The CD59 polypeptide used with the methods described herein can comprise or consist of an amino acid sequence at least about 80%, 85%, 90%, 95%, 97%, 98%, 99%, or 100% identical to that set forth in any one of SEQ ID NO: 1, SEQ ID NO: 2, or SEQ ID NO: 3. Additionally, the CD59 polypeptide used herein can comprise additional non-CD59 derived sequences, including purification tags, remnants of cleaved purification tags, additional complement inhibiting polypeptides, anti-inflammatory polypeptides, or polypeptides that increase stability or bioavailability of the CD59 polypeptide. Nucleic acids encoding a polypeptide that comprises or consists of a CD59 polypeptide, described herein, are also useful for the treatment of inflammasome mediated disorders. Thus, also envisioned is a nucleic acid or a plurality of nucleic acids that encode a polypeptide comprising or consisting of a CD59 polypeptide described herein. The nucleic acid can suitably be included in a recombinant vector such as a viral or plasmid vector that is suitable for administration to a subject. These vectors can additionally be included in pharmaceutical compositions for administration to an individual with an inflammasome mediated disorder.

A polypeptide comprising or consisting of the amino acid sequence set forth in any one of SEQ ID NO: 1, SEQ ID NO: 2, or SEQ ID NO: 3 can be used in a method of treating an inflammasome disorder in a subject as described herein. In certain embodiments, a polypeptide comprising or consisting of the amino acid sequence set forth in any one of SEQ ID NO: 1, SEQ ID NO: 2, or SEQ ID NO: 3 is used in a method of treating an inflammasome disorder in an eye of a subject. In certain embodiments, the inflammasome disorder is uveitis. In certain embodiments, the subject has displayed positive results for expression or activity of at least one inflammasome activity marker. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3). In certain embodiments, the methods described herein comprise administering a human CD59 polypeptide to an individual that has displayed positive results for expression or activity of at least one inflammasome activity marker. The CD59 polypeptide can comprise an amino acid sequence at least about 80%, 85%, 90%, 95%, 97%, 98%, 99%, or 100% identical to that set forth in any one of SEQ ID NO: 1, SEQ ID NO: 2, or SEQ ID NO: 3.

A nucleic acid or plurality of nucleic acids encoding a polypeptide comprising or consisting of the amino acid sequence set forth in any one of SEQ ID NO: 1, SEQ ID NO: 2, or SEQ ID NO: 3 can be used in a method of treating an inflammasome disorder in a subject. In certain embodiments, the nucleic acid or plurality of nucleic acids encoding a polypeptide comprising or consisting of the amino acid sequence set forth in any one of SEQ ID NO: 1, SEQ ID NO: 2, or SEQ ID NO: 3 is used in a method of treating an inflammasome disorder in an eye of a subject. In certain embodiments, the inflammasome disorder is uveitis. In certain embodiments, the subject has displayed positive results for expression or activity of at least one inflammasome activity marker. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3).

In certain embodiments, the methods described herein comprise administering a human CD59 polypeptide to an individual that has displayed positive results for expression or activity of at least one inflammasome activity marker. The CD59 polypeptide can comprise or consist of an amino acid sequence at least about 80%, 85%, 90%, 95%, 97%, 98%, 99%, or 100% identical to that set forth in any one of SEQ ID NO: 1, SEQ ID NO: 2, or SEQ ID NO: 3. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3). In certain embodiments, the positive results are obtained from the eye of the individual.

In certain embodiments, the methods described herein comprise administering a human CD59 polypeptide to an eye of an individual that has displayed positive results for expression or activity of at least one inflammasome activity marker. The CD59 polypeptide can comprise or consist of an amino acid sequence at least about 80%, 85%, 90%, 95%, 97%, 98%, 99%, or 100% identical to that set forth in any one of SEQ ID NO: 1, SEQ ID NO: 2, or SEQ ID NO: 3. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3). In certain embodiments, the positive results are obtained from the eye of the individual.

In certain embodiments, the methods described herein comprise administering a human CD59 polypeptide to an individual afflicted with uveitis. The CD59 polypeptide can comprise or consist of an amino acid sequence at least about 80%, 85%, 90%, 95%, 97%, 98%, 99%, or 100% identical to that set forth in any one of SEQ ID NO: 1, SEQ ID NO: 2, or SEQ ID NO: 3. In certain embodiments, the polypeptide is administered to an afflicted eye of the individual.

In certain embodiments, the methods described herein comprise administering a nucleic acid encoding a human CD59 polypeptide to an individual that has displayed positive results for expression or activity of at least one inflammasome activity marker. The nucleic acid can encode a CD59 polypeptide comprising or consisting of an amino acid sequence at least about 80%, 85%, 90%, 95%, 97%, 98%, or 99% identical to that set forth in SEQ ID NO: 1, SEQ ID NO: 2, or SEQ ID NO: 3. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3). In certain embodiments, the positive results are obtained from the eye of the individual.

In certain embodiments, the methods described herein comprise administering a nucleic acid encoding a human CD59 polypeptide to an eye of an individual that has displayed positive results for expression or activity of at least one inflammasome activity marker. The nucleic acid can encode a CD59 polypeptide comprising or consisting of an amino acid sequence at least about 80%, 85%, 90%, 95%, 97%, 98%, or 99% identical to that set forth in SEQ ID NO: 1, SEQ ID NO: 2, or SEQ ID NO: 3. In certain embodiments, the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+. In certain embodiments, the inflammasome activity marker is selected from: apoptosis-associated speck-like protein containing a CARD (PYCARD/ASC), or a NACHT, LRR and PYD domains-containing protein (NALP). In certain embodiments, the NALP is NACHT, LRR and PYD domains-containing protein 3 (NLRP3). In certain embodiments, the positive results are obtained from the eye of the individual.

In certain embodiments, the methods described herein, comprise administering a nucleic acid encoding a human CD59 polypeptide to an individual afflicted with uveitis. The nucleic acid can encode a CD59 polypeptide comprising or consisting of an amino acid sequence at least about 80%, 85%, 90%, 95%, 97%, 98%, or 99% identical to that set forth in SEQ ID NO: 1, SEQ ID NO: 2, or SEQ ID NO: 3. In certain embodiments, the nucleic acid is administered to an afflicted eye of the individual.

Calculations of sequence identity between sequences are performed as follows. To determine the percent identity of two amino acid sequences, the sequences are aligned for optimal comparison purposes (e.g., gaps can be introduced in one or both of a first and a second amino acid sequence for optimal alignment). The amino acid residues at corresponding amino acid positions or nucleotide positions are then compared. When a position in the first sequence is occupied by the same amino acid residue or nucleotide as the corresponding position in the second sequence, then the proteins are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences are accomplished using a mathematical algorithm. Percent identity between two amino acid sequences is determined using an alignment software program using the default parameters. Suitable programs include, for example, CLUSTAL W by Thompson et al., Nuc. Acids Research 22:4673, 1994 (www.ebi.ac.uk/clustalw), BL2SEQ by Tatusova and Madden, FEMS Microbiol. Lett. 174:247, 1999 (www.ncbi.nlm.nih.gov/blast/bl2seq/bl2.html), SAGA by Notredame and Higgins, Nuc. Acids Research 24: 1515, 1996 (igs-server.cnrs-mrs.fr/~cnotred), and DIALIGN by Morgenstern et al., Bioinformatics 14: 290, 1998 (bibiserv.techfak.uni-bielefeld.de/dialign).

### Vectors

The term "recombinant" refers to proteins produced by manipulation of genetically modified organisms, for example micro-organisms.

In accordance with the present invention a source of CD59 includes polynucleotide sequences that encode the CD59 protein, for example, engineered into recombinant DNA molecules to direct expression of the CD59 protein in appropriate host cells. To express a biologically active CD59 protein, a nucleotide sequence encoding the CD59 protein, or functional equivalent, is inserted into an appropriate expression vector, *i.e.*, a vector that contains the necessary nucleic acid encoding elements that regulate transcription and translation of the inserted coding sequence, operably linked to the nucleotide sequence encoding the CD59 protein amino acid sequence.

Methods that are well known to those skilled in the art are used to construct expression vectors containing a sequence encoding the CD59 protein operably linked to appropriate transcriptional and translational control elements. These methods include *in vitro* recombinant DNA techniques, synthetic techniques and *in vivo* recombination or genetic recombination. Such techniques are described in Sambrook et al., Molecular Cloning: A Laboratory Manual, Cold Spring Harbor Press, Plainview, NY, 1989, Molecular Biomethods Handbook, 2nd ed., J. M. Walker et al., Humana Press, 2008, and Handbook of Molec. and Cellul. Methods in Biol. and Med., 3rd ed., L. J. Ceske et al., CRC Press, 2011.

A variety of commercially available expression vector/host systems are useful to contain and express a CD59 protein encoding sequence. These include but are not limited to microorganisms such as bacteria transformed with recombinant bacteriophage, plasmid or cosmid DNA expression vectors; yeast transformed with yeast expression vectors; insect cell systems contacted with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti, pBR322, or pET25b plasmid); or animal cell systems. See Ausubel et al., Current Protocols in Molecular Biology, John Wiley & Sons, New York, NY, 1989, Molecular Biomethods Handbook, 2nd ed., J. M. Walker et al., Humana Press, 2008, and Handbook of Molec. and Cellul. Methods in Biol. and Med., 3rd ed., L. J. Ceske et al., CRC Press, 2011.

Virus vectors include, but are not limited to, adenovirus vectors, lentivirus vectors, adeno-associated virus (AAV) vectors, and helper-dependent adenovirus vectors. Virus vectors deliver a nucleic acid sequence that encodes CD59 protein that as shown herein interferes with the deleterious action of the MAC in pathogenesis of AMD. Adenovirus packaging vectors are commercially available from American Type Tissue Culture Collection (Manassas, VA). Methods of constructing adenovirus vectors and using adenovirus vectors are shown in Klein et al. 2007 Ophthalmology 114: 253-262, and van Leeuwen et al. 2003 Eur. J. Epidemiol. 18: 845-854.

Adenovirus vectors have been used in eukaryotic gene expression (Levrero et al. 1991 Gene, 101: 195-202) and vaccine development (Graham et al. 1991 Methods in Molecular Biology: Gene Transfer and Expression Protocols 7, (Murray, Ed.), Humana Press, Clifton, NJ, 109-128). Further, recombinant adenovirus vectors are used for gene therapy (Wu et al. U.S. patent number 7,235,391 issued June 26, 2007 which is incorporated herein by reference in its entirety).

Recombinant adenovirus vectors are generated, for example, from homologous recombination between a shuttle vector and a provirus vector (Wu et al., U.S. patent number 7,235,391 issued June 26, 2007; incorporated by reference). The adenovirus vectors herein are replication defective, for example, are conditionally defective, lacking adenovirus E1 region, and a polynucleotide encoding CD59 is introduced at the position from which the E1-coding sequences have been removed. The polynucleotide encoding the CD59 gene alternatively is inserted in the E3 region.

Helper cell lines may be derived from human cells such as, 293 human embryonic kidney cells, muscle cells, hematopoietic cells or other human embryonic mesenchymal or epithelial cells. Alternatively, the helper cells may be derived from the cells of other mammalian species that are permissive for human adenovirus, e.g., Vero cells or other monkey embryonic mesenchymal or epithelial cells. Generation and propagation of these replication defective adenovirus vectors using a helper cell line is described in Graham et al 1977 J. Gen. Virol. 36: 59-72.

Lentiviral vector packaging vectors are commercially available from Invitrogen Corporation (Carlsbad CA). An HIV-based packaging system for the production of lentiviral vectors is prepared using constructs in Naldini et al. 1996 Science 272: 263-267; Zufferey et al. 1997 Nature Biotechnol. 15: 871-875; and Dull et al. 1998 J. Virol. 72: 8463-8471.

A number of vector constructs are available to be packaged using a system, based on third-generation lentiviral SIN vector backbone (Dull et al. 1998 J. Virol. 72: 8463-8471). For example the vector construct pRRLsinCMVGFPpre contains a 5' LTR in which the HIV promoter sequence has been replaced with that of Rous sarcoma virus (RSV), a self-inactivating 3' LTR containing a deletion in the U3 promoter region, the HIV packaging signal, RRE sequences linked to a marker gene cassette consisting of the *Aequora* jellyfish green fluorescent protein (GFP) driven by the CMV promoter, and the woodchuck hepatitis virus PRE element, which appears to enhance nuclear export. The GFP marker gene allows quantitation of transfection or transduction efficiency by direct observation of UV fluorescence microscopy or flow cytometry (Kafri et al. 1997 Nature Genet. 17: 314-317; and Sakoda et al. 1999 J. Mol. Cell. Cardiol. 31: 2037-2047).

Manipulation of retroviral nucleic acids to construct a retroviral vector containing the gene that encodes for CD59 protein and packaging cells is accomplished using techniques known in the art. See Ausubel, et al., 1992, Volume 1, Section III (units 9.10.1-9.14.3); Sambrook, et al., 1989. Molecular Cloning: A Laboratory Manual. Second Edition. Cold Spring Harbor Laboratory Press, Cold Spring Harbor, N.Y.; Miller, et al., Biotechniques. 7:981-990, 1989; Molecular Biomethods Handbook, 2nd ed., J. M. Walker et al., Humana Press, 2008; Handbook of Molec. and Cellul. Methods in Biol. and Med., 3rd ed., L. J. Ceske et al., CRC Press, 2011; Eglitis, et al., Biotechniques. 6:608-614, 1988; U.S. patent numbers 4,650,764, 4,861,719, 4,980,289, 5,122,767, and 5,124,263; and PCT patent publications numbers WO 85/05629, WO 89/07150, WO 90/02797, WO 90/02806, WO 90/13641, WO 92/05266, WO 92/07943, WO 92/14829, and WO 93/14188, each of which is incorporated by reference in its entirety.

A retroviral vector is constructed and packaged into non-infectious transducing viral particles (virions) using an amphotropic packaging system. Examples of such packaging systems are found in, for example, Miller et al. 1986 Mol. Cell Biol. 6 :2895-2902; Markowitz et al. 1988 J. Virol. 62:1120-1124; Cosset et al. 1990 J. Virol. 64: 1070-1078; U.S. patent numbers 4,650,764, 4,861,719, 4,980,289, 5,122,767, and 5,124,263, and PCT patent publications numbers WO 85/05629, WO 89/07150, WO 90/02797, WO 90/02806, WO 90/13641, WO 92/05266, WO 92/07943, WO 92/14829, and WO 93/14188, each of which is incorporated by reference in its entirety.

Generation of "producer cells" is accomplished by introducing retroviral vectors into the packaging cells. Examples of such retroviral vectors are found in, for example, Korman et al. 1987 Proc. Natl. Acad. Sci. USA. 84: 2150-2154; Morgenstern et al. 1990 Nucleic Acids Res. 18: 3587-3596; U.S. patent numbers 4,405,712, 4,980,289, and 5,112,767; and PCT patent publications numbers WO 85/05629, WO 90/02797, and WO 92/07943; each of which is incorporated by reference.

Herpesvirus packaging vectors are commercially available from Invitrogen Corporation, (Carlsbad, CA). Exemplary herpesviruses are an α-herpesvirus, such as *Varicella-Zoster* virus or pseudorabies virus; a herpes simplex virus such as HSV-1 or HSV-2; or a herpesvirus such as Epstein-Barr virus. A method for preparing empty herpesvirus particles that can be packaged with a desired nucleotide segment, for example a CD59 nucleotide or polynucleotide sequence, in the absence of a helper virus that is capable to most herpesviruses is shown in Fraefel et al. (U.S. patent number 5,998,208, issued December 7, 1999 which is incorporated by reference in its entirety).

The herpesvirus DNA vector can be constructed using techniques familiar to the skilled artisan. For example, DNA segments encoding the entire genome of a herpesvirus is divided among a number of vectors capable of carrying large DNA segments, e.g., cosmids (Evans, et al., Gene 79, 9-20, 1989), yeast artificial chromosomes (YACS) (Sambrook, J. et al., MOLECULAR CLONING: A LABORATORY MANUAL, 2nd Edition, Cold Spring Harbor Press, Cold Spring Harbor, N.Y., 1989) or *E. coli* F element plasmids (O'Conner et al. 1989 Science 244:1307-1313).

For example, sets of cosmids have been isolated which contain overlapping clones that represent the entire genomes of a variety of herpesviruses including Epstein-Barr virus, *Varicella-Zoster* virus, pseudorabies virus and HSV-1. See M. van Zijl et al. 1988 J. Virol. 62: 2191; Cohen et al. 1993 Proc. Nat'l Acad. Sci. U.S.A. 90: 7376; Tomkinson et al. 1993 J. Virol. 67: 7298; and Cunningham et al. 1993 Virology 197: 116.

AAV is a dependent parvovirus in that it depends on co-infection with another virus (either adenovirus or a member of the herpes virus family) to undergo a productive infection in cultured cells (Muzyczka 1992 Curr Top Microbiol Immunol, 158: 97 129). For example, recombinant AAV (rAAV) virus is made by co-transfecting a plasmid containing the gene of interest, for example, the CD59 gene, flanked by the two AAV terminal repeats (McLaughlin et al. 1988 J. Virol., 62(6): 1963-1973; Samulski et al. 1989 J. Virol, 63: 3822-3828) and an expression plasmid containing the wild-type AAV coding sequences without the terminal repeats. Cells are also contacted or transfected with adenovirus or plasmids carrying the adenovirus genes required for AAV helper function. Recombinant AAV virus stocks made in such fashion include with adenovirus which must be physically separated from the recombinant AAV particles (for example, by cesium chloride density centrifugation).

Adeno-associated virus (AAV) packaging vectors are commercially available from GeneDetect (Auckland, New Zealand). AAV has been shown to have a high frequency of integration and infects non-dividing cells, thus making it useful for delivery of genes into mammalian cells in tissue culture (Muzyczka 1992 Curr Top Microbiol Immunol 158: 97-129). AAV has a broad host range for infectivity (Tratschin et al. 1984 Mol. Cell. Biol. 4: 2072-2081; Laughlin et al. 1986 J. Virol., 60(2): 515-524; Lebkowski et al. 1988 Mol. Cell. Biol. 8(10): 3988-3996; McLaughlin et al. 1988 J. Virol. 62(6): 1963-1973).

Methods of constructing AAV vectors and using AAV vectors are described, for example in U.S. patent numbers 5,139,941 (Wu et al.) issued June 26, 2007 and 4,797,368 (Carter et al.) issued January 10, 1989; each of which is incorporated by reference. Use of AAV in gene delivery is further described in LaFace et al. 1988 Virology 162(2): 483 486; Zhou et al. 1993 Exp. Hematol, 21: 928-933; Flotte et al. 1992 Am. J. Respir. Cell Mol. Biol. 7(3): 349-356; and Walsh et al. 1994 J. Clin. Invest 94: 1440-1448.

Recombinant AAV vectors have been used successfully for *in vitro* and *in vivo* transduction of marker genes (Kaplitt et al. 1994 Nat Genet., 8(2): 148-154; Lebkowski et al. 1988 Mol. Cell. Biol. 8(10): 3988-3996; Samulski et al. 1991 EMBO J. 10: 3941-3950; Shelling and Smith 1994 Gene Therapy, 1: 165-169; Yoder et al. 1994 Blood, 82 (Supp.): 1: 347A; Zhou et al. 1993 Exp. Hematol 21: 928-933; Tratschin et al. 1985 Mol. Cell. Biol. 5: 3258-3260; McLaughlin et al. 1988 J. Virol. 62(6): 1963-1973) and transduction of genes involved in human diseases (Flotte et al. 1992 Am. J. Respir. Cell Mol. Biol. 7(3): 349-356; Ohi et al. 1990 Gene, 89(2): 279-282; Walsh et al. 1994 J. Clin. Invest. 94: 1440-1448; and Wei et al. 1994 Gene Therapy, 1: 261 268).

In certain embodiments, the vectors herein are non-viral vectors for example synthetic gene delivery vehicles or vectors that are not related to a virus particle and that specifically deliver the CD59 gene encoding material to the target cells or tissue. Examples of non-viral vectors include liposomes, peptides, nanoparticles, emulsions, or encapsulated two or more phase systems or other suitable preparation. Thus, in certain embodiments, a method, kit, or composition involves a non-viral vector with nucleic acid that is loaded and contacted to a tissue or cell. For example a liposome containing naked DNA encoding a membrane-independent CD59 protein having a modified GPI anchor that does not target a membrane, or a gene encoding a membrane-independent CD59 protein having no GPI anchor, is encapsulated in the liposome and the liposome is contacted to the tissue or cell such that the nucleic acid is effectively delivered to the tissue or cell for treatment of a complement-related disease.

### Antibodies

The term "antibody" as referred to herein includes whole antibodies and any antigen binding fragment (i.e., "antigen-binding portion") or single chains of these. A naturally occurring "antibody" is a glycoprotein including at least two heavy (H) chains and two light (L) chains inter-connected by disulfide bonds.

As used herein, an antibody that "specifically binds to human MAC" is intended to refer to an antibody that binds to human MAC with a K_{D} of 5 × 10⁻⁹ M or less, 2 × 10⁻⁹ M or less, or 1 × 10⁻¹⁰ M or less. For example, the antibody is monoclonal or polyclonal. The terms "monoclonal antibody" or "monoclonal antibody composition" as used herein refer to a preparation of antibody molecules of single molecular composition. A monoclonal antibody composition displays a single binding specificity and affinity for MAC or for a particular epitope of MAC. The antibody is an IgM, IgE, IgG, such as IgG1 or IgG4.

Also useful for MAC assay is an antibody that is a recombinant antibody. The term "recombinant human antibody", as used herein, includes all antibodies that are prepared, expressed, created or isolated by recombinant means, such as antibodies isolated from an animal (e.g., a mouse). Mammalian host cells for expressing the recombinant antibodies used in the methods herein include Chinese Hamster Ovary (CHO cells) including dhfr- CHO cells, described in Urlaub and Chasin 1980 Proc. Natl. Acad. Sci. USA 77: 4216-4220, and used with a DH FR selectable marker, e.g., as described in R.J. Kaufman and P.A. Sharp, 1982 Mol. Biol. 159:601-621, NSO myeloma cells, COS cells and SP2 cells. In particular, for use with NSO myeloma cells, another expression system is the GS gene expression system shown in WO 87/04462, WO 89/01036 and EP 338,841. To produce antibodies, expression vectors encoding intact or a portion of the protein of interest are introduced into mammalian host cells, and the host cells are cultured for a period of time sufficient to allow for expression of the antibody in the host cells or secretion of the antibody into the culture medium in which the host cells are grown. Antibodies can be recovered from the culture medium using standard protein purification methods.

Standard assays to evaluate the binding ability of the antibodies toward the target of various species are known in the art, including for example, ELISAs, western blots and RIAs. The binding kinetics (e.g., binding affinity) of the antibodies also can be assessed by standard assays known in the art, such as by Biacore analysis.

General methodologies for antibody production, including criteria to be considered when choosing an animal for the production of antisera, are described in Harlow et al. (1988 Antibodies, Cold Spring Harbor Laboratory, pp. 93-117). For example, an animal of suitable size such as goats, dogs, sheep, mice, or camels are immunized by administration of an amount of immunogen, such as the intact protein or a portion thereof containing an epitope from human MAC, effective to produce an immune response. An exemplary protocol is as follows. The animal is subcutaneously injected in the back with 100 micrograms to 100 milligrams of antigen, dependent on the size of the animal, followed three weeks later with an intraperitoneal injection of 100 micrograms to 100 milligrams of immunogen with adjuvant dependent on the size of the animal, for example Freund's complete adjuvant. Additional intraperitoneal injections every two weeks with adjuvant, for example Freund's incomplete adjuvant, are administered until a suitable titer of antibody in the animal's blood is achieved. Exemplary titers include a titer of at least about 1:5000 or a titer of 1:100,000 or more, i.e., the dilution having a detectable activity. The antibodies are purified, for example, by affinity purification on columns containing human MAC.

The technique of *in vitro* immunization of human lymphocytes is used to generate monoclonal antibodies. Techniques for *in vitro* immunization of human lymphocytes are well known to those skilled in the art. See, e.g., Inai et al. May 1993 Histochemistry, 99(5): 335-362; Mulder et al. 1993 Hum. Immunol. 36(3): 186-192; Harada, et al. 1993 J. Oral Pathol. Med., 22(4): 145-152; Stauber, et al. 1993 J. Immunol. Methods 161(2): 157-168; and Venkateswaran, et al. 1992 Hybridoma, 11(6): 729-739. These techniques can be used to produce antigen-reactive monoclonal antibodies, including antigen-specific IgG, and IgM monoclonal antibodies. Any antibody or fragment thereof having affinity and specific for human MAC is within the scope of the assay for MAC deposition provided herein.

In examples herein, contacting with CD59 is achieved by injecting cells or tissues with a vector encoding the CD59 gene.

In examples herein, cell lysis is measured by propidium iodide (PI) uptake. PI is commercially available from, for example, Fluka BioChemica (Buchs, Switzerland). PI is an intercalating agent that fluoresces when bound to DNA. PI is membrane impermeant and generally excluded from viable cells, thus PI is commonly used to identify and/or determine the amount of non-living cells in a mixed population.

In examples herein and in certain embodiments, the detectable protein is a fluorescent protein, for example, green fluorescent protein, aequorin, cyan fluorescent protein, DsRed fluorescent protein, enhanced green fluorescent protein, and yellow fluorescent protein. Green fluorescent protein (GFP) and aequorin are bioluminescent compositions isolated from the jellyfish *Aequorea victoria.* When a calcium ion binds to aequorin, the complex breaks down into apoaequorin and a luminescent composition, which emits blue light. Synthetic aequorin is commercially available from Sealite, Sciences (Bogart, Ga.) as AQUALITE^{®}. GFP emits light in the lower green portion of the visible spectrum, and synthetic GFP is commercially available from Clontech (Mountain View, CA).

Mutations to the amino acid sequence of GFP have been made to produce derivative amino acid sequences of GFP that fluoresce different colors, for example, cyan fluorescent protein, DsRed fluorescent protein, enhanced green fluorescent protein, and yellow fluorescent protein. Synthetic cyan fluorescent protein, synthetic DsRed fluorescent protein, synthetic enhanced green fluorescent protein, and synthetic yellow fluorescent protein are each commercially available from Clontech (Mountain View, CA).

In alternative embodiments, the detectable agent is a fluorescent agent that is not a fluorescent protein, for example, Indocyanine Green, Doxorubicin, Riboflavin, Chlorophyll, and Porphyrin.

Indocyanine Green (ICG) is a tricarbocyanine dye that upon excitation, emits lights at about 800nm, about 820nm, about 840nm or at about 860nm. ICG is commercially available from H.W. Sands Corp. (Jupiter, FL). Doxorubicin is fluorescent and emits light at wavelengths of, for example, about 550nm, 600nm, or 650nm. Doxorubicin is commercially available from Sigma-Aldrich (St. Louis, MO). Riboflavin is commercially available from Sigma-Aldrich (St. Louis, MO) and is fluorescent, emitting light at a wavelength of, for example, about 450nm, about 550nm, about 650nm, or about 750nm. Chlorophyll A is a green photosynthetic pigment that emits light at a wavelength of, for example, about 600nm, about 700nm, or about 800nm. Chlorophyll A is commercially available from suppliers such as Sigma Chemical (St. Louis, MO) and Turner Designs (Sunnyvale, CA). Porphyrin is a heterocyclic macrocycle made from 4 pyrrole subunits linked on opposite sides through 4 methine bridges (=CH-). The extensive conjugated structure of Porphyin makes the compound chromatic, i.e., fluorescent at a wavelength of, for example, about 600nm, or about 650nm, or about 700nm. Porphyrin is commercially available from Sigma-Aldrich (St. Louis, MO).

In other alternative embodiments, the detectable agent is an enzymatic agent, which is a protein, for example, β-galactosidase or alkaline phosphatase, that can be expressed on a nucleotide vector.

β-galactosidase is a hydrolase enzyme that catalyzes the hydrolysis of β-galactosides into monosaccharides. A luminescent β-galactosidase detection kit is commercially available from Clontech (Mountain View, CA). Alkaline phosphatase is a hydrolase enzyme responsible for removing phosphate groups from many types of molecules, including nucleotides, proteins, and alkaloids. A luminescent alkaline phosphatase detection kit is commercially available from Sigma Aldrich (St. Louis, MO).

### Pharmaceutical compositions

An aspect of the present invention provides a method using a nucleic acid encoding a CD59 protein for inhibiting inflammasome activation in cells of an inflammation-affected eye in a subject, or a soluble CD59 protein. In various embodiments, the CD59 protein includes a membrane-independent (soluble) CD59 protein. In certain embodiments, the CD59 composition is formulated for intravenous administration. In certain embodiments, the composition is compounded as an ophthalmologic formulation for administration to the eye and may be compounded to enhance delivery to the fundus, to provide sustained release locally at the retina or otherwise formulated to provide effective treatment of the vessels and/or tissue involved in ocular diseases including macular degeneration. In related embodiments, the pharmaceutical composition is formulated sufficiently pure for administration to a human subject, e.g., to the circulation or eye of a human subject. In certain embodiments, these compositions optionally further include one or more additional therapeutic agents. In certain embodiments, the additional therapeutic agent or agents are selected from the group consisting of growth factors, anti-inflammatory agents, vasopressor agents including but not limited to nitric oxide and calcium channel blockers, collagenase inhibitors, topical steroids, matrix metalloproteinase inhibitors, ascorbates, angiotensin II, angiotensin III, calreticulin, tetracyclines, fibronectin, collagen, thrombospondin, transforming growth factors (TGF), keratinocyte growth factor (KGF), fibroblast growth factor (FGF), insulin-like growth factors (IGFs), IGF binding proteins (IGFBPs), epidermal growth factor (EGF), platelet derived growth factor (PDGF), neu differentiation factor (NDF), hepatocyte growth factor (HGF), vascular endothelial growth factor (VEGF), heparin-binding EGF (HBEGF), thrombospondins, von Willebrand Factor-C, heparin and heparin sulfates, and hyaluronic acid.

In other embodiments, the additional agent is a compound, composition, biological or the like that potentiates, stabilizes or synergizes or even substitutes for the ability of CD59 protein to protect cells from MAC deposition. Also included are therapeutic agents that may beneficially or conveniently be provided at the same time as the CD59 protein, such as agents used to treat the same, a concurrent or a related symptom, condition or disease. In some embodiments, the drug may include without limitation anti-tumor, antiviral, antibacterial, anti-mycobacterial, anti-fungal, anti-proliferative or anti-apoptotic agents. Drugs that are included in the compositions of the invention are well known in the art. See for example, Goodman & Gilman's The Pharmacological Basis of Therapeutics, 9th Ed., Hardman, et al., eds., McGraw-Hill, 1996, the contents of which are herein incorporated by reference herein.

As used herein, the term "pharmaceutically acceptable carrier" includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences Ed. by Gennaro, Mack Publishing, Easton, PA, 1995 provides various carriers used in formulating pharmaceutical compositions and known techniques for the preparation thereof. Some examples of materials which can serve as pharmaceutically acceptable carriers include, but are not limited to, sugars such as glucose and sucrose; excipients such as cocoa butter and suppository waxes; oils such as peanut oil, cottonseed oil, safflower oil, sesame oil, olive oil, corn oil, and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, preservatives and antioxidants can also be present in the composition, according to the judgment of the formulator.

### Therapeutically effective dose

Treatment of an inflammation affected eye by methods provided herein involves contacting a tissue or cells with a pharmaceutical composition, for example, administering a therapeutically effective amount of a pharmaceutical composition having as an active agent a nucleic acid encoding a CD59 protein or a source of expression of a CD59 protein, to a subject in need thereof, in such amounts and for such time as is necessary to achieve the desired result. Methods for example include treating uveitis by contacting an ocular tissue or cell with a CD59 protein or with a vector encoding the CD59 protein.

The compositions, according to the method of the present invention, may be administered using any amount and any route of administration effective for treating uveitis or other complement-related diseases and conditions. Thus, the expression "amount effective for treating uveitis", as used herein, refers to a sufficient amount of composition to beneficially prevent or ameliorate the symptoms of uveitis.

The exact dosage is chosen by the individual physician in view of the patient to be treated. Dosage and administration are adjusted to provide sufficient levels of the active agent(s) or to maintain the desired effect. Additional factors which may be taken into account include the severity of the disease state, e.g., intermediate or advanced stage of uveitis; age, weight and gender of the patient; diet, time and frequency of administration; route of administration; drug combinations; reaction sensitivities; and tolerance/response to therapy. Long acting pharmaceutical compositions might be administered hourly, twice hourly, every 3 to four hours, daily, twice daily, every 3 to 4 days, every week, or once every two weeks depending on half-life and clearance rate of the particular composition.

The active agents of the invention are preferably formulated in dosage unit form for ease of administration and uniformity of dosage. The expression "dosage unit form" as used herein refers to a physically discrete unit of active agent appropriate for the patient to be treated. It will be understood, however, that the total daily usage of the compositions of the present invention will be decided by the attending physician within the scope of sound medical judgment. For any active agent, the therapeutically effective dose can be estimated initially either in cell culture assays or in animal models, as provided herein, usually mice, but also potentially from rats, rabbits, dogs, or pigs. The animal cell model provided herein is also used to achieve a desirable concentration and total dosing range and route of administration. Such information can then be used to determine useful doses and routes for administration in humans.

A therapeutically effective dose refers to that amount of active agent that ameliorates the symptoms or condition or prevents progression of uveitis. Therapeutic efficacy and toxicity of active agents can be determined by standard pharmaceutical procedures in cell cultures or experimental animals, e.g., ED50 (the dose is therapeutically effective in 50% of the population) and LD50 (the dose is lethal to 50% of the population). The dose ratio of toxic to therapeutic effects is the therapeutic index, and it can be expressed as the ratio, LD50/ED50. Pharmaceutical compositions which exhibit large therapeutic indices are preferred. The data obtained from cell culture assays and animal studies are used in formulating a range of dosage for human use.

The daily dosage of the products may be varied over a wide range, such as from 0.001 to 100 mg per adult human per day. For ocular administration, the compositions are preferably provided in the form of a solution containing 0.001, 0.01, 0.05, 0.1, 0.5, 1.0, 2.5, 5.0, 10.0, 15.0, 25.0, 50.0, 100.0, 250.0, or 500.0 micrograms of the active ingredient for the symptomatic adjustment of the dosage to the patient to be treated.

The dose of a soluble CD59 protein, administered intravenously, can be in a range from 0.01 milligrams per kilogram per dose to about 10 milligrams per kilogram per dose, from 0.1 milligrams per kilogram per dose to about 5 milligrams per kilogram per dose, or from 1.0 milligrams per kilogram per dose to about 5 milligrams per kilogram per dose. The dosing protocol includes a frequency of daily, twice a week, once a week, once every two weeks, once every three weeks, or once every four weeks.

A unit dose typically contains from about 0.001 micrograms to about 500 micrograms of the active ingredient, preferably from about 0.1 micrograms to about 100 micrograms of active ingredient, more preferably from about 1.0 micrograms to about 10 micrograms of active ingredient. An effective amount of the drug is ordinarily supplied at a dosage level of from about 0.0001 mg/kg to about 25 mg/kg of body weight per day. For example, the range is from about 0.001 to 10 mg/kg of body weight per day, or from about 0.001 mg/kg to 1 mg/kg of body weight per day. The compositions may be administered on a regimen of, for example, one to four or more times per day. A unit dose may be divided for example, administered in two or more divided doses.

Administration as a source of expression of a CD59 protein is administration of a dose of a viral vector or a nucleic acid vector, such that the dose contains at least about 50, 100, 500, 1000, or at least about 5000 particles per cell to be treated. Cell number can be calculated from retinal area in need of treatment by methods known to one of skill in the art of treating uveitis or eye disorders.

### Administration of pharmaceutical compositions

As formulated with an appropriate pharmaceutically acceptable carrier in a desired dosage, the pharmaceutical composition provided herein is administered to humans and other mammals topically such as ocularly (as by solutions, ointments, or drops), nasally, bucally, orally, rectally, parenterally, intracisternally, intravaginally, or intraperitoneally.

Ocular injections include intra-ocular injection into the aqueous or the vitreous humor, or injection into the external layers of the eye, such as via subconjunctival injection or subtenon injection.

Liquid dosage forms for ocular, oral, intravenous, or other systemic administration include, but are not limited to, pharmaceutically acceptable emulsions, microemulsions, solutions, suspensions, syrups and elixirs. In addition to the active agent(s), the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the ocular, oral, or other systemically-delivered compositions can also include adjuvants such as wetting agents, and emulsifying and suspending agents.

Dosage forms for topical or transdermal administration of an inventive pharmaceutical composition include ointments, pastes, creams, lotions, gels, powders, solutions, sprays, inhalants, or patches. The active agent is admixed under sterile conditions with a pharmaceutically acceptable carrier and any needed preservatives or buffers as may be required. For example, ocular or cutaneous routes of administration are achieved with aqueous drops, a mist, an emulsion, or a cream. Administration may be therapeutic or it may be prophylactic. The invention includes ophthalmological devices, surgical devices, audiological devices or products, which contain disclosed compositions (e.g., gauze bandages or strips), and methods of making or using such devices or products. These devices may be coated with, impregnated with, bonded to or otherwise treated with a composition as described herein.

Transdermal patches have the added advantage of providing controlled delivery of the active ingredients to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of inj ectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. In order to prolong the effect of an active agent, it is often desirable to slow the absorption of the agent from subcutaneous or intramuscular injection. Delayed absorption of a parenterally administered active agent may be accomplished by dissolving or suspending the agent in an oil vehicle. Injectable depot forms are made by forming microencapsule matrices of the agent in biodegradable polymers such as polylactide-polyglycolide. Depending upon the ratio of active agent to polymer and the nature of the particular polymer employed, the rate of active agent release can be controlled. Examples of other biodegradable polymers include poly(orthoesters) and poly(anhydrides). Depot injectable formulations are also prepared by entrapping the agent in liposomes or microemulsions which are compatible with body tissues.

Compositions for rectal or vaginal administration are preferably suppositories which can be prepared by mixing the active agent(s) of this invention with suitable non-irritating excipients or carriers such as cocoa butter, polyethylene glycol or a suppository wax which are solid at ambient temperature but liquid at body temperature and therefore melt in the rectum or vaginal cavity and release the active agent(s).

Solid dosage forms for oral administration include capsules, tablets, pills, powders, and granules. In such solid dosage forms, the active agent is mixed with at least one inert, pharmaceutically acceptable excipient or carrier such as sodium citrate or dicalcium phosphate and/or a) fillers or extenders such as starches, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof.

Solid compositions of a similar type may also be employed as fillers in soft and hard-filled gelatin capsules using such excipients as milk sugar as well as high molecular weight polyethylene glycols and the like. The solid dosage forms of tablets, dragees, capsules, pills, and granules can be prepared with coatings and shells such as enteric coatings, release controlling coatings and other coatings well known in the pharmaceutical formulating art. In such solid dosage forms the active agent(s) may be admixed with at least one inert diluent such as sucrose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active agent(s) only, or preferentially, in a certain part of the intestinal tract, optionally, in a delayed manner. Examples of embedding compositions which can be used include polymeric substances and waxes.

A portion of this work, attached hereto as Appendix A, is prepared for publication as a manuscript entitled, "Complement mediated activation of the NLRP3 Inflammasome and its Inhibition by AAV mediated delivery of CD59 in a model of uveitis" by co-authors and co-inventors Binit Kumar, Siobhan M. Cashman and Rajendra Kumar-Singh, which is hereby incorporated by reference herein in its entirety.

### Disease monitoring and selection of patients for treatment

The CD59 nucleic acid and protein compositions, described herein, are useful for the treatment of inflammatory and inflammasome related diseases. The compositions described can be administered to an individual selected for treatment on the basis of a positive result for a test that assays for inflammasome activity. Such a test is not restricted to direct measures of inflammasome activation, such as but also to indirect measures. An indirect measure can be for instance measurement of cytokines, chemokines, cell-surface markers of inflamed or diseased tissues, cell activation of specific immune types, presence or absence of specific immune types. For example, cytokines such as IL-1α, IL-1β and IL-18 are elevated in response to inflammasome activation. Therefore, the individual selected may display elevated levels of these cytokines in plasma, serum, blood, histological sections, mRNA or cell extracts of a diseased tissue, or elevated levels of expression or secretion by immune cells isolated from the blood or a diseased tissue. A selection criteria for treatment with a CD59 nucleic acid or protein composition can be elevated levels or activity of any one or more of caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.

Elevated levels of cytokines IL-1α, IL-1β, IL-β17, IL-18, or IFN-γ, can be assessed, for example, by ELISA, AlphaLISA^{®}, immunohistochemistry, flow cytometry analysis of relevant cell populations, or quantitative RT-PCR. Caspase and inflammasome activation can be determined by immunohistochemistry of a biopsy specimen using antibodies specific for activated caspase and inflammasome components, such as caspase 1, caspase 5, apoptosis-associated speck-like protein containing a CARD (PYCARD/ASC), a NACHT, LRR and PYD domains-containing protein (NALP). Th1 and Th17 cells can be identified using flow cytometry for cell surface markers CXCR3 or CCR5 or for the transcription factor TBX21, in the case of Th1; or for cell surface markers CCR6 andCCR4, or intracellular staining for the transcription factor RORg/gt in the case of Th17.

The CD59 nucleic acid and protein compositions described can be administered to an individual selected for treatment on the basis of a diagnosis of an inflammasome related disorder such as Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis. Included are inflammasome related eye disorders such as a uveitis, an allergic conjunctivitis, a blepharitis, a chronic conjunctivitis, an episcleritis, a keratitis, a retinitis, an ocular cicatricle pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.

In certain aspects the CD59 nucleic acid or protein compositions can be administered after an individual has been monitored for a response to at least one dose of such a composition or another treatment to reduce inflammation or resolve an inflammasome related disease. This cycle of administration and monitoring can be repeated 1, 2, 3, 4, 5, 6, 7, 8, 9 or more times or until an adequate clinical response is achieved or until inflammasome activity has been reduced. In certain embodiments, the CD59 nucleic acid and protein compositions can be: 1) administered to an individual selected as having an inflammasome related disease or a positive result for inflammasome activity; 2) the individual can then be monitored and on the basis of worsening, no change, or a sub-optimal response as determined by a test for inflammasome activity or diagnostic criteria for an inflammasome related disease, the individual can receive at least one or more subsequent administrations of the composition. In certain embodiments, the CD59 nucleic acid and protein compositions can be: 1) administered to an individual; 2) the individual can then be monitored and on the basis of worsening, no change, or a sub-optimal or greater response as determined by a test for inflammasome activity or diagnostic criteria for an inflammasome related disease, the individual can receive at least one or more subsequent administrations of the composition.

The following numbered embodiments encompassing the methods and compositions of the CD59 polypeptides/nucleic acids are envisioned herein:
1. A method for inhibiting inflammasome activation in cells of an inflammation-affected eye in a subject, the method comprising, administering to the subject a composition comprising a nucleotide sequence encoding a membrane independent CD59 protein operably linked to a promoter for expression and secretion of the membrane independent CD59 protein in the cells of the inflammation-affected eye, the composition inhibiting inflammasome activation.
2. The method according to embodiment 1, wherein the composition inhibits inflammasome activation independent of function of Membrane Attack Complex (MAC).
3. The method according to embodiment 1, wherein the subject has at least one condition selected from: a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.
4. The method according to embodiment 3, wherein the uveitis is at least one selected from: anterior uveitis, intermediate uveitis, posterior uveitis, and panuveitis.
5. The method according to embodiment 1 further comprising obtaining a sample from the subject.
6. The method according to embodiment 5, wherein the sample is at least one selected from: tears, blood, urine, eye discharge, phlegm, and mucus.
7. The method according to any of embodiments 1-6 further comprising before administering, measuring in the eye at least one of a retinal function of the eye and an inflammasome activity marker.
8. The method according to any of embodiments 1-7 further comprising after administering, measuring in the eye at least one of a retinal function of the eye and an inflammasome activity marker.
9. The method according to embodiment 7 or 8, wherein the inflammasome activity marker is at least one selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
10. The method according to embodiment 7 or 8, wherein measuring the retinal function or the inflammasome activity marker further comprises performing at least one procedure selected from: an eye exam, an Optical Coherence Tomography (OCT), a Conjunctival Impression Cytology (CIT), an RTPCR, an ELISA and a PCR.
11. The method according to embodiment 3 further comprising administering the composition in a dosage sufficient to treat the uveitis.
12. The method according to embodiment 1 further comprising prior to administering, engineering the nucleotide sequence in a viral vector.
13. The method according to embodiment 1 or 12 further comprising administering the nucleotide sequence as a naked nucleic acid.
14. The method according to embodiment 13, wherein the viral vector is a genetically engineered genome of at least one virus selected from the group consisting of: an adenovirus, an adeno-associated virus, a herpesvirus, and a lentivirus.
15. The method according to embodiment 14, wherein the lentivirus is a retrovirus.
16. The method according to embodiment 1 further comprising engineering the membrane independent CD59 protein to have at least one mutation resulting in loss of function of a glycosyl phosphatidyl inositol (GPI) anchoring domain of the translated CD59 protein.
17. The method according to embodiment 1, further comprising engineering nucleotide sequence encoding the membrane independent CD59 protein by deleting nucleotides encoding the region of the glycosyl phosphatidyl inositol (GPI) anchoring domain.
18. The method according to embodiment 1, wherein administering further comprises injecting the composition ocularly.
19. The method according to embodiment 1, wherein administering further comprises applying the composition topically.
20. The method according to embodiment 18, wherein the ocular injection is selected from the group consisting of subretinal injection, vitreous injection, intra-ocular injection, subconjunctival injection, and subtenon injection.
21. The method according to embodiment 18, wherein ocular injecting further comprises administering to an external layer of the eye.
22. The method according to embodiment 1, further comprising administering an additional therapeutic agent to the eye.
23. The method according to embodiment 22, wherein the additional therapeutic agent is at least one selected from the group consisting of: anti-tumor, antiviral, antibacterial, anti-mycobacterial, anti-fungal, anti-proliferative and anti-apoptotic.
24. The method according to embodiment 22, wherein the additional therapeutic agent is selected from the group consisting of: a growth factor, an antiinflammatory agent, a vasopressor agent, a collagenase inhibitor, a steroid, a matrix metalloproteinase inhibitor, an ascorbate, an angiotensin, a calreticulin, a tetracycline, a fibronectin, a collagen, a thrombospondin, a transforming growth factors (TGF), a keratinocyte growth factor (KGF), a fibroblast growth factor (FGF), an insulin-like growth factors (IGFs), an IGF binding protein (IGFBP), an epidermal growth factor (EGF), a platelet derived growth factor (PDGF), a neu differentiation factor (NDF), a hepatocyte growth factor (HGF), a vascular endothelial growth factor (VEGF), a heparin-binding EGF (HBEGF), a thrombospondin, a von Willebrand Factor-C, a heparin, a heparin sulfate, and a hyaluronic acid.
25. A kit for inhibiting an activity of an inflammasome in an inflammation-affected eye in a subject, the kit comprising:
   a. a pharmaceutical composition comprising a membrane-independent CD59 protein and/or a nucleotide sequence encoding the CD59 protein, wherein the composition is in a dosage sufficient to inhibit the inflammasome in the inflammation-affected eye in the subject;
   b. instructions for use; and,
   c. a container.
26. A kit for treating uveitis in a subject comprising:
   a. a pharmaceutical composition comprising a membrane-independent CD59 protein and/or a nucleotide sequence encoding the CD59 protein, wherein the composition is in a dosage sufficient to treat the uveitis in the subject;
   b. instructions for use; and,
   c. a container.
27. A method for treating at least one condition selected from: a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a polyarteritis nodosa, a Wegener's granulomatosis a Vogt-Koyanagi-Harada Disease, a sympathetic ophthalmia, and a sarcoidosis, the method comprising administering to the subject a composition comprising:
   a) a nucleotide sequence encoding a membrane independent CD59 protein operably linked to a promoter for expression and secretion of the membrane independent CD59 protein in the cells of the inflammation-affected eye; or
   b) a membrane independent CD59 protein;
      wherein the composition inhibits inflammasome activation.
28. A method for inhibiting inflammasome activation in cells of an inflammation-affected eye in a subject, the method comprising:
   a) measuring in the eye an inflammasome activity marker; and
   b) if the inflammasome activity marker is positive administering to the subject a composition comprising a nucleotide sequence encoding a membrane independent CD59 protein operably linked to a promoter for expression and secretion of the membrane independent CD59 protein in the cells of the inflammation-affected eye, the composition inhibiting inflammasome activation.
29. The method according to embodiment 28, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
30. The method according to embodiment 28 or 29, further comprising measuring in the eye an inflammasome activity marker after the administering of the composition comprising the nucleotide sequence encoding the membrane independent CD59 protein operably linked to the promoter for the expression and secretion of the membrane independent CD59 protein.
31. A method for inhibiting inflammasome activation in the cells of an inflammation-affected subject, the method comprising:
   a. administering to the subject a composition comprising a nucleotide sequence encoding a membrane independent CD59 protein operably linked to a promoter for expression and secretion of the membrane independent CD59 protein in the cells of the inflammation-affected subject; or
   b. administering to the subject a composition comprising a soluble CD59 protein;
   wherein the composition inhibits inflammasome activation.
32. The method according to embodiment 31, wherein the composition inhibits inflammasome activation independent of function of Membrane Attack Complex (MAC).
33. The method according to embodiment 31, wherein the subject has at least one inflammasome associated condition selected from: Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis.
34. The method according to embodiment 31, further comprising obtaining a sample from the subject.
35. The method according to embodiment 34, wherein the sample is at least one selected from: blood, plasma, serum, peripheral blood mononuclear cells, cerebrospinal fluid, and urine.
36. The method according to any of embodiments 31-35, further comprising before administering, measuring in the subject an inflammasome activity marker.
37. The method according to any of embodiments 31-35, further comprising after administering, measuring in the subject an inflammasome activity marker.
38. The method according to embodiment 36 or 37, wherein the inflammasome activity marker is at least one selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
39. The method according to embodiment 38, further comprising administering the composition in a dosage sufficient to treat the inflammasome associated condition.
40. The method according to embodiment 39, wherein the nucleotide sequence encoding a membrane independent CD59 protein operably linked to a promoter for expression and secretion of the membrane independent CD59 protein is carried in a vector comprising a genetically engineered genome of at least one virus selected from the group consisting of: an adenovirus, an adeno-associated virus, a herpesvirus, and a lentivirus.
41. The method according to embodiment 40, wherein the lentivirus is a retrovirus.
42. The method according to embodiment 31, wherein administering further comprises injecting the composition intravenously.
43. The method according to embodiment 31, wherein administering further comprises applying the composition topically.
44. The method according to embodiment 31, further comprising administering an additional therapeutic agent.
45. The method according to embodiment 44, wherein the additional therapeutic agent is at least one selected from the group consisting of: anti-tumor, antiviral, antibacterial, anti-mycobacterial, anti-fungal, anti-proliferative and anti-apoptotic.
46. The method according to embodiment 44, wherein the additional therapeutic agent is selected from the group consisting of: a growth factor, an antiinflammatory agent, a vasopressor agent, a collagenase inhibitor, a steroid, a matrix metalloproteinase inhibitor, an ascorbate, an angiotensin, a calreticulin, a tetracycline, a fibronectin, a collagen, a thrombospondin, a transforming growth factors (TGF), a keratinocyte growth factor (KGF), a fibroblast growth factor (FGF), an insulin-like growth factors (IGFs), an IGF binding protein (IGFBP), an epidermal growth factor (EGF), a platelet derived growth factor (PDGF), a neu differentiation factor (NDF), a hepatocyte growth factor (HGF), a vascular endothelial growth factor (VEGF), a heparin-binding EGF (HBEGF), a thrombospondin, a von Willebrand Factor-C, a heparin, a heparin sulfate, and a hyaluronic acid.
47. A kit for inhibiting an activity of an inflammasome in an inflammation-affected cell in a subject, the kit comprising:
   c. a pharmaceutical composition comprising:
      i. a membrane-independent CD59 protein and/or a nucleotide sequence encoding the CD59 protein; or
      ii. a soluble CD59 protein; wherein the composition is in a dosage sufficient to inhibit the inflammasome in the inflammation-affected cell in the subject;
   d. instructions for use; and
   e. a container.
48. A method for treating at least one condition selected from: Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, and a sarcoidosis, the method comprising:
   administering to the subject a composition comprising a nucleotide sequence encoding a membrane independent CD59 protein operably linked to a promoter for expression and secretion of the membrane independent CD59 protein from cells; or, a soluble CD59 protein; the composition inhibiting inflammasome activation.

The invention having now been fully described is further illustrated by the following examples and claims, which are illustrative and are not meant to be further limiting.

### Examples

### Example 1: Mice

C57BL/6J and C9-/- mice in the C57BL/6J background were purchased from The Jackson Laboratory (Bar Harbor, ME) and maintained in the animal facilities at Tufts University School of Medicine, Boston. All animal study protocols conformed to the Association for Research in Vision and Ophthalmology resolution on the use of Animals in Vision Research and the recommendations of the National Institutes of Health Guide for the Care and Use of Laboratory Animals.

### Example 2: AAV constructs and intravitreal injections

An AAV serotype 2 vector expressing a truncated form of human CD59 or protectin (AAVCAGsCD59) in which the glycosyl- phosphatidylinositol (GPI) anchoring signal has been deleted was constructed by protocols described in Cashman, S. M., et al. (2011) PLoS One 6, e19078. The soluble CD59 (sCD59) is expressed from a chicken β-actin promoter. As a negative control, a similar vector expressing green fluorescent protein (AAVCAGGFP) was used. Six-week-old C57Bl/6J mice were injected with 3.5 × 10⁹ genome copies/µl of AAVCAGsCD59 or AAVCAGGFP or PBS (1 µl) and one week later the mice were challenged with EAU as described in examples herein.

### Example 3: Induction of EAU by Active Immunization

Six-week-old C57Bl/6J and C9-/- mice in the same genetic background were immunized with 200 µg of human interphotoreceptor retinoid-binding protein (IRBP) peptide 1-20 (amino acid sequence: GPTHLFQPSLVLDMAKVLLD, SEQ ID NO: 1; Biomatik Corporation, Cambridge, ON, Canada) emulsified in 200 µl of 1:1 vol/vol with Complete Freund's adjuvant (CFA) containing *Mycobacterium tuberculosis* strain H37RA (2.5 mg/mL). The mice simultaneously received 1.5 µg *Bordetella pertussis* toxin diluted in 100ul PBS via an intraperitoneal injection (Agarwal, R. K., et al. (2012) Methods Mol. Biol. 900, 443-469).

### Example 4: Immunohistochemistry

Cryostat retinal sections (10 µm) were rehydrated in PBS for 15 min, blocked with 6% normal goat serum in PBS for one hour and incubated overnight in a moist chamber with the primary antibody against Rabbit Anti-Human C5b-9 (Complement Technology, Inc., Tyler, TX; dilution: 1:800, diluted in PBS containing 2% normal goat serum). Subsequently, sections were washed and incubated in anti-rabbit secondary antibody conjugated to Cy3 (Molecular Probes, Eugene, OR) to localize C5b-9 in retinal sections. Slides were mounted in anti-fade medium containing DAPI (Vectashield-DAPI; Vector Laboratories, Burlingame, CA) to counterstain the nuclei and images were obtained with a Leica confocal microscope. The intensity of C5b-9 staining in the entire section was quantified using Imaged software (National Institutes of Health; Bethesda, MD).

### Example 5: Western Blot Analysis

Murine retinas were harvested and homogenized in ice-cold RIPA buffer containing 50 mM Tris-HCl (pH 7.4), 250 mM NaCl, and 1% Nonidet P-40, with a protease inhibitor cocktail. Each protein sample (35 µg) was separated by sodium dodecyl sulfate polyacrylamide gel electrophoresis (Any kD^{™} Mini-PROTEAN^{®} precast gel, Biorad, CA) for NLRP3, Caspase-1, and ASC, and then transferred onto nitrocellulose membranes. After blocking with blocking buffer (LI-COR, Lincoln, NE, USA), mixed with 0.1% Tween-20, Immunoblots were incubated overnight at 4°C with mouse anti-NLRP3 monoclonal, mouse anti-Caspase1 monoclonal and rabbit anti-ASC polyclonal (Adipogen Corporation, San Diego, CA; dilution: 1:500) as the primary antibodies. Following incubation with the appropriate secondary antibody, the immunoreactive bands were visualized using LI-COR-Odyssey infra-red scanner (LI-COR). The blots were re-probed with β-actin as a loading control.

### Example 6: Enzyme-Linked Immunosorbent Assay

The cytokines were quantified by sandwich ELISA for mouse IL-1 β, IFN-γ and IL-17 (PeproTech, Rocky Hills, NJ), as per the manufacturer's instructions using 20 µg retinal protein supernatant. Supernatants were added in duplicate, and the cytokine being measured was revealed with a monoclonal antibody conjugated to horseradish peroxidase. The concentration of cytokines was described in pg/mL.

### Example 7: Gene expression

Total RNA was isolated from mouse retina using the RNeasy mini kit (QIAGEN, Valencia, CA) according to the manufacturer's protocol. RNA was quantified by 260 nm absorbance in a 'Nanodrop' and 1 µg RNA was used for cDNA synthesis using the High Capacity cDNA reverse transcription kit (Applied Biosystems, Foster City, CA).
Real-time polymerase chain reaction (RT-PCR) was performed using predesigned TaqMan primers for beta-actin (Mm02619580_g1), IL-1β (Mm00434228_m1), IL-17(Mm00439619_m1) and IFN-γ (Mm01168134_m1). Denaturation was performed at 95°C for 10 min, followed by 40 cycles at 95°C for 15 seconds, and annealing and extension were performed at 60°C for 60 seconds. The final PCR products were electrophoresed on a 2% agarose gel to confirm PCR specificity. The Ct values obtained from the RT-PCR were normalized to the Ct value from b-actin in the same sample using the ddCt method and fold-change data in gene expression was obtained. Gene expression for IL-17 was quantified semi-quantitatively.

### Example 8: Flow cytometry

Draining lymph node cells were isolated from mice and single cell suspensions were obtained by passing the cells through a 40µm nylon mesh. Draining lymph node cells that had been stimulated with 50 ng/mL PMA and 500 ng/mL ionomycin (Sigma-Aldrich, St Louis, MO) for four hours in the presence of GolgiStop (BD Biosciences, San Jose, CA) before intracellular cytokine staining. Cells were stained for the surface markers CD4 and then fixed with Cytofix/Cytoperm Buffer (BD Biosciences, San Jose, CA) for cytokine staining. Cells were stained with appropriately diluted fluorophore-labeled antibodies for intracellular targets (IFNγ, IL-17; eBiosciences, Inc., San Diego, CA) and respective isotype controls in Perm/Wash buffer (BD Biosciences, San Jose, CA). Flow cytometry was performed on a FACS Calibur (BD Biosciences), and data were analyzed with FlowJo software (Tree Star, Ashland, OR). Gates were set based on appropriate isotype controls. Where indicated, the percentage of positive cells represents the percentage in the gated population relative to normal controls.

### Example 9: Electroretinogram

Scotopic and photopic Electroretinogram (ERG) analysis was used to measure the loss of rod and cone function. Three weeks after EAU induction ERG examinations were recorded using a UTAS system with BigShot ganzfeld (LKC Technologies; Gaithersburg, MD). Following dark adaptation overnight, mice were anesthetized with ketamine (100 mg/kg)/xylazine (10 mg/kg) intraperitoneal injection under dark conditions. The pupils were dilated with 1% tropicamide and 2.5% phenylephrine hydrochloride. ERG active contact lens gold electrodes were placed gently on the center of cornea with a drop of lubricant (GenTeal, Alcon, Inc., Fort Worth, TX) to maintain corneal hydration and better electrical conductivity. Reference electrodes and ground electrodes were inserted subcutaneously in the back of the neck and near the tail base, respectively. Scotopic ERGs were elicited with 10 msec flashes of white light at 0 dB (5 cds/m²), -10 dB (25 cds/m²), -20 dB (25 cds/m²). Simultaneous, photopic ERGs were examined after a two-minute white light bleach. The photopic responses were elicited with flashes of white light at 0 dB and 1dB (3.15 cds/m²) intensity. Ten responses were averaged at each flash intensity. The amplitude of the a-wave was measured from the baseline to the negative peak of a-wave, and the b-wave was measured from the negative peak of the a-wave to the peak of the b-wave.

### Example 10: Spectral Domain Optical Coherence Tomography (OCT) and Fundus imaging

Mice were anesthetized with a ketamine and xylazine cocktail and pupils were dilated with a drop of 1% tropicamide and 2.5% phenylephrine. Corneas were kept moistened by topical application of an eye lubricant (GenTeal, Alcon, Inc., Fort Worth, TX). Optical Coherence Tomography (OCT) images were acquired using a Bioptigen Spectral Domain Ophthalmic Imaging System (Bioptigen Envisu R2300, Morrisville, NC). Averaged single B scan and volume scans were obtained with images centered on the optic nerve head as described in Chen, J., et al. (2013) PLoS One 8, e63904.

After 24 days of EAU, fundus imaging was performed and images were captured using a Micron III Retinal Imaging Microscope and StreamPix software (Phoenix Research Labs, Pleasanton, CA). Specifically, eyes were examined for physiological and pathological symptoms such as infiltrates, cuffing around the blood vessels, white linear lesions, retinal dystrophy, subretinal hemorrhages and retinal detachment. An individual score was evaluated by two independent observers in a blinded fashion on a scale of 0-4 for each of the individual parameters: retinal infiltrates, optic disc changes, vascularity, and structural damage. Clinical scores were calculated by averaging the score for each of these four criteria (Xu, H., et al. (2008) Exp. Eye Res. 87, 319-326).

### Example 11: Histopathology

Eyes for histologic examination from all groups were harvested at 24 days post immunization and fixed in 10% buffered formalin. After fixation for two days, specimens were dehydrated by graded alcohol steps and embedded in paraffin blocks. Six vertical sections (5 µm) were cut at six different planes including the optic nerve region and stained with hematoxylin and eosin. The detailed severity of EAU was assessed on a scale of 0-4 for photoreceptor damage, infiltration and vasculitis as described previously in Caspi, R. R., et al. (1988) J. Immunol 140, 1490-1495. Based on these criteria, the photoreceptor damage score was calculated as a combined score of photoreceptor loss, retinal folds and retinal detachment. Similarly, the infiltration score was comprised of a combination of granuloma, hemorrhage, DF Nodule and infiltrates. Vasculitis score represents perivascular inflammation and CD4 cells infiltration around vasculature, formation of thrombi and extent of vasculature affected in the retina.

### Example 12: Statistical Analysis

Results are shown as mean ± SEM. Mann-Whitney test was used for a clinical score, histology score and FACS analysis. Statistical differences between two groups were analyzed using an unpaired t-test. For comparison between more than two groups, one-way ANOVA was performed. A p-value of less than or equal to 0.05 was considered statistically significant.

### Example 13: MAC Deposition in EAU

A final step in the activation of complement results in the deposition of the MAC on the surface of cells. Recruitment of multiple C9 molecules into the preformed C5b-8 complex is the final and necessary step in the assembly of the MAC (C5b-9). Mice deficient in C9 are thus unable to form functional MAC complexes.

In order to determine whether MAC is formed on the surface of retinal cells in EAU frozen retinal sections were examined at 24 days post induction of EAU in C57Bl/6J mice by staining with antibody against C5b-9. Deposition of MAC on the retina of EAU mice was observed to be 70% greater compared to MAC deposition on the retina of normal control mice. MAC deposition on the surface of C9-/- EAU retina was observed to be not significant (Figure 1A and Figure 9). The data obtained indicated that complement is activated in EAU and that the reaction reaches completion to form MAC on retinal tissues. The data indicates also that C9-/- EAU mice were unable to form MAC on the retina.

### Example 14: Activation of the Inflammasome in EAU

Deposition of MAC on the surface of cells leads to the formation of a pore which results in an increase in intracellular calcium. In examples herein it was examined whether deposition of MAC triggered activation of the NLRP3 inflammasome and subsequent secretion of IL-1β.

The data obtained found that EAU in C57Bl/6J mice led to 112% greater IL-1 β protein and a corresponding 45 fold greater IL-1β mRNA than controls of normal mice. In contrast, C9-/- EAU mice were observed to achieve 37% greater IL-1β protein and a corresponding 3.8 fold greater IL-1β mRNA (Figure 1B and C). Western blot analyses indicated a 200% greater NLRP3 protein in EAU mice, and C9-/- EAU mice had 8% greater NLRP3 protein (Figure 1D).

An important component of the inflammasome complex is caspase-1, which generally occurs in an inactive zymogen form, activated by NLRP3 complex by proteolysis into active p10 and p20 subunits to make the final multimeric inflammasome complex. Western blot analyses yielded an 80% greater activation of caspase-1 (p20) in retinas of EAU mice than normal; however, 25% greater activation of caspase-1(p20) was observed in retinas of C9-/- EAU mice compared to normal (Figure 1E). The expression of ASC protein which is an inflammasome adapter protein in retinas of EAU mice was measured by western blot. Western blot analyses indicated that 44% greater ASC protein was observed in EAU mice, and 18% greater ASC protein was observed in C9-/-EAU mice than normal (Figure 1F).

Therefore, each of II,1- β, NLRP3, Caspase1, and ASC were observed to be elevated in EAU, and C9-/- EAU mice were partially protected from such increase, which indicates that deposition of MAC is an important player in the activation of the inflammasome in EAU.

### Example 15: Role of the MAC on Retinal Function in EAU

The retinal function in EAU and C9-/- EAU mice was measured using Electroretinogram (ERG). EAU mice retinal function was observed to have dark adapted a-wave amplitudes that were reduced by 33%, 50% and 41% at flash intensities of -20dB, -10dB and OdB respectively compared to control C57Bl/6J mice. C9-/- EAU mice retinal function was observed to have a-wave amplitudes that less, specifically by 9%, 40% and 27% at flash intensities of -20dB, -10dB and OdB respectively than normal control C57Bl/6J mice (Figure 2A and Figure 2B).

Dark-adapted b-wave amplitudes in EAU mice were observed to be reduced by 47%, 52% and 49% at flash intensities of -20dB, -10dB and OdB respectively, compared to control C57Bl/6J mice. C9-/- EAU mice were observed to have dark adapted b-wave amplitudes that were less, specifically by 32%, 33% and 17% at flash intensities of -20dB, -10dB and OdB respectively, compared to control C57Bl/6J mice. Although differences were observed in both a wave and b wave dark-adapted ERGs, the OdB was observed to be statistically (p<0.05) significant (Figure 2A and Figure 2B).

EAU mice were observed to have light adapted b-wave amplitudes that were reduced by 44% and 37% at flash intensities of 0dB and 1dB respectively, compared to control C57Bl/6J mice. C9-/- EAU mice were observed to have b-wave amplitudes that were reduced by 5% and 15% at flash intensities of 0dB and 1dB respectively, compared to control C57Bl/6J mice. Although differences were observed in light adapted b wave amplitudes, the OdB was observed to be statistically (p<0.05) significant (Figure 2A and Figure 2B).

### Example 16: No Significant Preservation of Retinal Structure in C9^{-/-} EAU

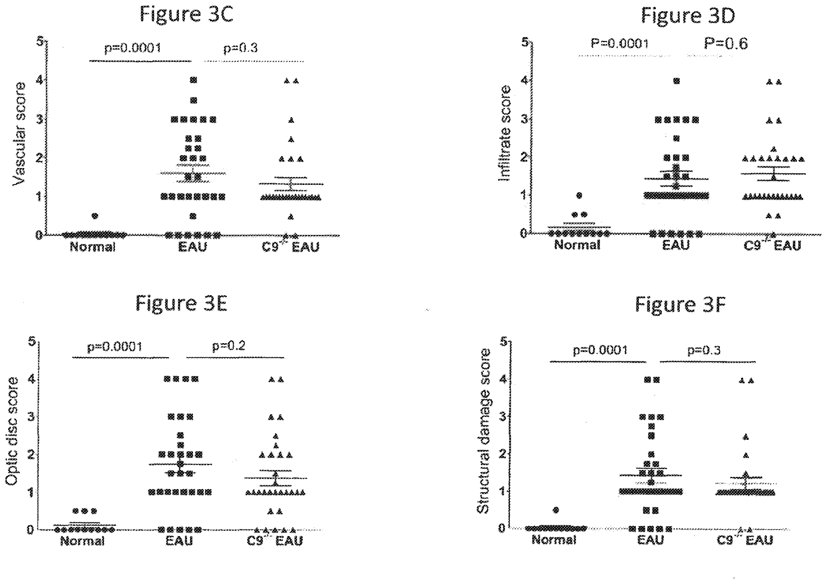

The retinal structure and pathological severity of disease in EAU and C9-/- EAU mice was quantified using fundus imaging, OCT, and histology at 24 days post induction of EAU. Based on clinical scoring criteria developed by Xu, H., et al. (2008) Exp. Eye Res. 87, 319-326, fundus imaging indicated that EAU mice had severe inflammation specifically an overall clinical score 16 fold greater than the control group, including a 38 fold greater vascular inflammation, 8 fold greater infiltration of immune cells, 13 fold greater damage to the optic disc and a 34 fold greater structural damage compared to control C57Bl/6J mice. Unexpectedly, C9-/- mice undergoing uveitis were observed to have pathological outcomes statistically equivalent to EAU mice (Figure 3).

Twenty-four days post immunization, EAU mice were observed to exhibit a severe inflammatory cellular infiltration into the vitreous and choroid, retinal vasculitis, retinal edema and a moderate to severe retinal folding and infiltrates compared to C57Bl/6J control mice as observed by OCT imaging and histopathology (Figure 4A and Figure 4B). A detailed histological analysis was performed in paraffin embedded sections from control, EAU and C9-/- EAU mice retinas and scored based on criteria described in Caspi, R. R., et al. (1988) J. Immunol 140, 1490-1495. EAU mice were observed to have at least about 27 fold more infiltrates (Figure 4C), increased vasculitis (Figure 4D), and 78 fold greater photoreceptor damage (Figure 4D) than C57Bl/6J control mice. Although the histological score in C9-/- EAU mice had 28% less infiltration, 35% lower photoreceptor damage and 31% reduced vasculitis compared to EAU mice, the differences were not observed to be statistically significant (Figure-4B and C).

The photoreceptor damage score was calculated based on criteria described in Caspi, R. R., et al. (1988) J. Immunol 140, 1490-1495 as a combined score of photoreceptor loss, retinal folds and retinal detachment. Similarly, the infiltration score was comprised of a combination of granuloma, hemorrhage, DF Nodule and infiltrates. Vasculitis score represents perivascular inflammation and CD4 cells infiltration around vasculature, formation of thrombi and extent of vasculature affected in the retina.

### Example 17: Soluble CD59 Mediated Inhibition of MAC Deposition in EAU

The data obtained in examples herein indicate that C9^{-/-} mice were unable to form MAC due to a genetic defect in C9 are also unable to activate the inflammasome and are protected from some of the features of uveitis. However, the retina of C9^{-/-}EAU mice was observed to be not protected from the histological pathology associated with EAU.

CD59 is a GPI-anchored protein found on the membrane of most nucleated cells. The major function of CD59 is to prevent the recruitment of C9 into the preformed C5b-8 complex. A recombinant adeno-associated virus vector expressing a truncated CD59 (AAVCAGsCD59) that has its GPI anchor signal deleted, enabling it to be secreted and diffuse throughout the retina was described in Cashman, S. M., et al. (2011) PLoS One 6, e19078.

Mice were injected intravitreally with AAVCAGsCD59 and one week later (to allow for optimal levels of transgene expression) the mice were challenged with EAU as described in the examples herein. For negative controls mice were intravitreally injected with AAVCAGGFP- a virus expressing green fluorescent protein (GFP) that were similarly challenged with EAU. At 24 days post induction of EAU, the frozen retinal sections from both sets of mice were examined by staining with C5b-9 antibody. Intravitreal injection of AAVCAGGFP in mice that were subsequently challenged with EAU led to transgene expression in the ganglion cell layer, inner plexiform layer and inner nuclear layer (Figure 14B). In mice with phenotypically more severe EAU, transgene expression could also be observed in the RPE and photoreceptors (Figure 14B, bottom row). AAVCAGsCD59-injected EAU mice were observed to have approximately 45% less deposition of MAC relative to AAVCAGGFP-injected EAU mice (Figure 5A and Figure 12). Therefore, intravitreally injected AAVCAGsCD59 was observed to inhibit the formation of MAC in the retina of EAU mice.

### Example 18: Soluble CD59 Mediated Inhibition of the Inflammasome in EAU

In examples herein, C9-/- EAU mice were observed to have significant reduction of activation of the inflammasome compared to control C57Bl/6J mice undergoing EAU. NLRP3/Caspase-1 mediated secretion of IL-1β in EAU mice that were pre-injected with either AAVCAGsCD59 or AAVCAGGFP was measured as described examples herein.

AAVCAGsCD59-injected EAU mice were observed to have 40% less IL-1β protein and 70% less IL-1β mRNA compared to AAVCAGsCD59-injected EAU mice as measured by ELISA and RT-PCR (Figure 5B and Figure 5C). Similarly, NLRP3 protein as well as Caspase 1 p20 levels were reduced by 60% in AAVCAGsCD59-injected EAU mice compared to control AAVCAGGFP-injected EAU mice (Figure 5D and Figure 5E). Substantial difference between the amounts of ASC in AAVCAGsCD59-injected EAU mice compared to control AAVCAGGFP-injected EAU mice was not observed (Figure 5F). Therefore, the data obtained indicate that sCD59 inhibits NLRP3 mediated IL-1β production by inhibiting MAC deposition in uveitis.

### Example 19: Role of the MAC mediated NLRP3 inflammasome activation in T cell Differentiation in EAU

CD4+ infiltrating T cells enter the retina in either a differentiated state or in an undifferentiated state into Th1 which are IFN-γ producing cells and Th17 which are IL-17 producing cells. To examine the potential role of the MAC in T cell differentiation, the levels of IFN-γ and IL-17 in EAU and C9-/- EAU mice retinas respectively were measured.

Using ELISA, a 102% amount of IFN-γ protein in EAU retinas was observed compared to control C57Bl/6J retinas. In contrast, 14% less IFN-γ protein was observed in C9^{-/-} EAU retinas compared to control C57Bl/6J retinas. RT-PCR data indicated a more than 200-fold and 14-fold increase, respectively, in IFN-γ mRNA in EAU and C9^{-/-} EAU retinas, respectively, compared to control C57Bl/6J retinas (Figure 10A and Figure 10B). Similarly, expression of IL-17 protein was observed to be 99% greater in EAU retinas compared to control C57Bl/6J retinas however expression of IL-17 protein was observed to be only 44% greater in C9^{-/-} EAU retinas compared to control C57Bl/6J retinas. Statistical difference between the expression levels of IL-17 mRNA was not observed in EAU and C9^{-/-} EAU retinas. IL-17 mRNA levels were observed to remain below the detection limit in C57Bl/6J retinas (Figure 10C and Figure 10D). These data indicate that MAC plays a role in T cell differentiation in EAU.

The levels of Th1 CD4+ cells and Th17 CD4+ cells were measured in draining lymph nodes (DLN) at 24 days post-EAU in control C57Bl/6J, EAU, and C9^{-/-} EAU mice. A greater number of IL-17 and IFN-γ positive CD4+ cells were observed in DLNs of EAU mice compared to control C57Bl/6J mice (Figure 11A- Figure 11C). A statistically significant difference in the levels of IFN-γ and IL-17 positive CD4+ cells in DLNs between EAU and C9^{-/-} EAU mice was not observed (Figure 11A- Figure 11C). These data indicate that MAC plays a significant role in T cell differentiation in the retina in EAU and that MAC may not play a significant role in DLNs.

### Example 20: The Impact of Soluble CD59 on T Cell Differentiation in EAU

To investigate the potential effects on T cell differentiation in sCD59-expressing eyes undergoing EAU, the levels of IFN-γ and IL-17 protein and mRNA in AAVCAGsCD59-injected EAU mice and AAVCAGGFP-injected EAU mice were measured.

The levels of IFN-γ and IL-17 protein were significantly lower in amount by more than 25% and 35% in AAVCAGsCD59-injected EAU retinas compared to control AAVCAGGFP-injected EAU retinas (Figure 13A and Figure 13C). Further, the levels of IFN-γ and IL-17 mRNA in freshly isolated AAVCAGsCD59-injected EAU retinas were observed to be 47% and 10% lower respectively compared to control AAVCAGGFP-injected EAU retinas. The differences in mRNA were not observed to be statistically significant (Figure 13B and Figure 13D).

### Example 21: Soluble CD59 Mediated Preservation of Retinal Function in EAU

To determine whether sCD59 could preserve retinal function in EAU, ERGs were performed in AAVCAGsCD59-injected or AAVCAGGFP-injected mice undergoing uveitis. AAVCAGsCD59 injected EAU mice were observed to have dark adapted larger a-wave amplitudes by 45%, 49% and 51% at flash intensities of -20dB, -10dB and OdB respectively compared to control AAVCAGGFP-injected mice. Similarly, the dark-adapted b-wave amplitudes in AAVCAGsCD59 injected mice were observed to be larger by 55%, 48% and 40% at -20dB, -10dB and OdB flashes intensities, respectively, compared to control AAVCAGGFP injected EAU control eyes (Figure 6). Further, the light adapted b-wave amplitude at OdB and 1dB flash intensity in AAVCAGsCD59 injected EAU eyes were preserved by 12% and 39% compared to control AAVCAGGFP injected control EAU eyes (Figure 6). A significant potentially therapeutic role was observed for recombinant sCD59 in preserving retinal function in the inner and outer retina in uveitis.

### Example 22: Soluble CD59 Mediated Preservation of Retinal Structure and Pathology in EAU

To determine whether sCD59 could preserve retinal structure and reduce pathology associated with uveitis, fundus imaging of AAVCAGsCD59 injected or AAVCAGGFP-injected EAU mice was performed. Based on scoring criteria described by Xu, H., et al. (2008) Exp. Eye Res. 87, 319-326 it was determined that the overall clinical score in AAVCAGsCD59 injected EAU mice was 22% less compared to control AAVCAGGFP injected EAU mice. The overall clinical score included of the appearance of 24% fewer inflammatory infiltrates, 27% less structural damage, 22% reduced vasculitis and cuffing of vessels, and 13% less damage to the optic disc in AAVCAGsCD59 -injected EAU mice compared to AAVCAGGFP-injected EAU mice (Figure 7A- Figure7F). Significant difference was not observed between PBS vehicle control and AAVCAGGFP -injected EAU retinas. Each of vascular, infiltration and structural damage scores were observed to be statistically significantly different (p<0.05), however, the optic disc score did not reach statistical significance (p=0.1). Therefore, the data obtained indicate that expression of sCD59 reduced retinal inflammation and infiltration of immune cells in uveitis.

The EAU progression and severity was observed to be greater in AAVCAGGFP-injected EAU mice compared to AAVCAGsCD59 as recorded and observed in OCT scans (Figure 8A). Detailed histological analysis of paraffin sections taken from the retina of AAVCAGsCD59-injected EAU mice or control AAVCAGGFP-injected EAU mice were performed. Similarly, based on criteria described by Caspi, R. R., et al. (1988) J. Immunol 140, 1490-1495, AAVC AGsCD59-injected EAU mice were observed to have approximately 76% fewer infiltrates, 69% less photoreceptor damage and 78% less vasculitis compared to control AAVCAGGFP-injected EAU mice (Figure 8B-Figure 8E). Photoreceptor damage and infiltration differences were observed to be statistically significant (p=0.03 and p=0.01 respectively), however, the vasculitis score was not statistically significant (p=0.2).

### Example 23: Treatment of an individual with atherosclerosis using a soluble CD59 protein composition and subsequent monitoring

An individual is diagnosed with atherosclerotic vascular disease based upon ultrasonography indicating an increased intimal medial thickness (IMT), the individual is then administered with a composition comprising a soluble CD59 protein by intravenous infusion. The patient receives three subsequent doses on a once a week basis. After dosing the individual is reassessed by ultrasonography, indicating no change in IMT the individual then receives an additional four doses of CD59 protein intravenously on a weekly basis followed by reassessment. At the follow up IMT thickness is reduced to an acceptable range and no further CD59 treatments are administered.

### Example 24: Treatment of an individual with sarcoidosis using a soluble CD59 protein composition and subsequent monitoring

An individual presents to her primary care physician with symptoms consistent with sarcoidosis, a lung X-ray shows growths consistent with such a diagnosis, a biopsy sample is taken, and elevated levels of activated caspase-1 are determined by comparison to a healthy tissue control. The individual is then administered with a composition comprising a soluble CD59 protein by intravenous infusion. After dosing the individual is reassessed by X-ray, indicating a partial reduction of the sarcoidosis. The patient is administered a soluble CD59 protein composition a second time, reassessment by X-ray indicates a complete response and no further CD59 treatments are administered.

As used herein the term "individual," "patient," or "subject" refers to individuals diagnosed with, suspected of being afflicted with, or at-risk of developing at least one disease for which the described compositions and method are useful for treating. In certain embodiments the individual is a mammal. In certain embodiments, the mammal is a mouse, rat, rabbit, dog, cat, horse, cow, sheep, pig, goat, llama, alpaca, or yak. In certain embodiments, the individual is a human.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention.

All publications, patent applications, issued patents, and other documents referred to in this specification are herein incorporated by reference as if each individual publication, patent application, issued patent, or other document was specifically and individually indicated to be incorporated by reference in its entirety. Definitions that are contained in text incorporated by reference are excluded to the extent that they contradict definitions in this disclosure.

The present application and invention further includes the subject matter of the following numbered clauses:
1. A method for inhibiting inflammasome activation in cells of an inflammation-affected eye in a subject, the method comprising: administering to the subject a composition comprising a nucleotide sequence encoding a membrane independent CD59 protein operably linked to a promoter for expression and secretion of the membrane independent CD59 protein in the cells of the inflammation-affected eye, wherein the composition inhibits inflammasome activation, wherein the subject has displayed positive results for expression or activity of at least one inflammasome activity marker in the inflammation-affected eye of the subject.
2. The method according to clause 1, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
3. The method according to clause 1, wherein the inflammasome activity marker is selected from: apoptosis-associated speck-like protein containing a CARD (PYCARD/ASC), or a NACHT, LRR and PYD domains-containing protein (NALP).
4. The method according to clause 3, wherein the NALP is NACHT, LRR and PYD domains-containing protein 3 (NLRP3).
5. The method according to any one of clauses 1 to 4, further comprising measuring in the eye an inflammasome activity marker after the administering of the composition comprising the nucleotide sequence encoding the membrane independent CD59 protein operably linked to the promoter for the expression and secretion of the membrane independent CD59 protein.
6. The method according to any one of clauses 1 to 5, wherein the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.
7. The method according to clause 6, wherein the subject has been diagnosed with or is suspected of being afflicted with a uveitis.
8. The method according to any one of clauses 1 to 7, wherein the positive results for expression or activity of at least one inflammasome activity marker in the inflammation-affected eye of the subject is elevated expression or activity of at least one inflammasome activity marker in comparison to expression or activity of at least one inflammasome activity marker in the eye of an individual not diagnosed or afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a polyarteritis nodosa, a Wegener's granulomatosis a Vogt-Koyanagi-Harada Disease, a sympathetic ophthalmia, or a sarcoidosis.
9. The method according to clause 8, wherein the positive results for expression or activity of at least one inflammasome activity marker in the inflammation-affected eye of the subject is elevated expression or activity of at least one inflammasome activity marker in comparison to expression or activity of at least one inflammasome activity marker in the eye of an individual not diagnosed or afflicted with a uveitis
10. The method according to any one of clauses 1 to 9, wherein the positive results for expression or activity of at least one inflammasome activity marker in the inflammation-affected eye of the subject is determined by a histological score.
11. A method for inhibiting inflammasome activation in cells of an inflammation-affected subject, the method comprising:
   a. measuring in the subject an inflammasome activity marker; and
   b. if the inflammasome activity marker is positive administering to the subject a composition comprising:
      i. a nucleotide sequence encoding a membrane independent CD59 protein operably linked to a promoter for expression and secretion of the membrane independent CD59 protein in the cells of the inflammation-affected subject; or
      ii. a soluble CD59 protein; the composition inhibiting inflammasome activation.
12. The method according to clause 11, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
13. The method according to clause 11, wherein the inflammasome activity marker is selected from: apoptosis-associated speck-like protein containing a CARD (PYCARD/ASC), or a NACHT, LRR and PYD domains-containing protein (NALP).
14. The method according to clause 13, wherein the NALP is NACHT, LRR and PYD domains-containing protein 3 (NLRP3).
15. The method according to any one of clauses 11 to 14, wherein the inflammasome activity marker is measured in an eye of the subject.
16. The method according to any one of clauses 11 to 15, further comprising measuring in the subject an inflammasome activity marker after the administering of the composition comprising the nucleotide sequence encoding the membrane independent CD59 protein operably linked to the promoter for the expression and secretion of the membrane independent CD59 protein or the soluble CD59 protein.
17. The method according to any one of clauses 11 to 16, wherein the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.
18. The method according to clause 17, wherein the subject has been diagnosed with or is suspected of being afflicted with a uveitis.
19. The method according to any one of clauses 11 to 18, wherein the positive inflammasome activity marker is elevated expression or activity of at least one inflammasome activity marker in comparison to expression or activity of at least one inflammasome activity marker in a subject not diagnosed or afflicted with Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, uveitis, or a sarcoidosis.
20. The method according to clause 19, wherein the positive inflammasome activity marker is elevated expression or activity of at least one inflammasome activity marker in comparison to expression or activity of at least one inflammasome activity marker in a subject not diagnosed with uveitis.
21. The method according to any one of clauses 11 to 20, wherein the positive inflammasome activity marker in the inflammation-affected subject is determined by a histological score.
22. A method for inhibiting inflammasome activation in cells of an inflammation-affected subject, by administering to the subject a composition comprising:
   a. a nucleotide sequence encoding a membrane independent CD59 protein operably linked to a promoter for expression and secretion of the membrane independent CD59 protein in the cells of the inflammation-affected subject, wherein the composition inhibits inflammasome activation; or
   b. a soluble CD59 protein;
   wherein the subject has displayed positive results for expression or activity of at least one inflammasome activity marker in the inflammation-affected subj ect.
23. The method according to clause 22, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
24. The method according to clause 22, wherein the inflammasome activity marker is selected from: apoptosis-associated speck-like protein containing a CARD (PYCARD/ASC), or a NACHT, LRR and PYD domains-containing protein (NALP).
25. The method according to clause 24, wherein the NALP is NACHT, LRR and PYD domains-containing protein 3 (NLRP3).
26. The method according to any one of clauses 22 to 25, wherein the positive results for expression or activity of at least one inflammasome activity marker in the inflammation-affected subject is in an eye of the inflammation-affected subj ect.
27. The method according to any one of clauses 22 to 26, wherein the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.
28. The method according to clause 27, wherein the subject has been diagnosed with or is suspected of being afflicted with a uveitis.
29. The method according to any one of clauses 22 to 28, wherein the positive results for expression or activity of at least one inflammasome activity marker in the inflammation-affected subject is elevated expression or activity of at least one inflammasome activity marker in comparison to expression or activity of at least one inflammasome activity marker in a subject not diagnosed or afflicted with Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, uveitis, or a sarcoidosis.
30. The method according to clause 29, wherein the positive results for expression or activity of at least one inflammasome activity marker in the inflammation-affected subject is elevated expression or activity of at least one inflammasome activity marker in comparison to expression or activity of at least one inflammasome activity marker in a subject not diagnosed with uveitis.
31. The method according to any one of clauses 22 to 30, wherein the positive results for expression or activity of at least one inflammasome activity marker in the inflammation-affected subject is determined by a histological score.
32. A method for inhibiting inflammasome activation in cells of a uveitis afflicted subject, by administering to the subject a composition comprising:
   a. a nucleotide sequence encoding a membrane independent CD59 protein operably linked to a promoter for expression and secretion of the membrane independent CD59 protein in the cells of the inflammation-affected subject, wherein the composition inhibits inflammasome activation; or
   b. a soluble CD59 protein.
33. The method according to clause 32, wherein the subject has displayed positive results for expression or activity of at least one inflammasome activity marker in an eye of the inflammation-affected subject.
34. The method according to clause 33, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
35. The method according to clause 33, wherein the inflammasome activity marker is selected from: apoptosis-associated speck-like protein containing a CARD (PYCARD/ASC), or a NACHT, LRR and PYD domains-containing protein (NALP).
36. The method according to clause 35, wherein the NALP is NACHT, LRR and PYD domains-containing protein 3 (NLRP3).
37. The method according to any one of clauses 33 to 36, wherein the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.
38. The method according to clause 37, wherein the subject has been diagnosed with or is suspected of being afflicted with a uveitis.
39. The method according to any one of clauses 33 to 38, wherein the positive results for expression or activity of at least one inflammasome activity marker in the inflammation-affected subject is elevated expression or activity of at least one inflammasome activity marker in comparison to expression or activity of at least one inflammasome activity marker in a subject not diagnosed or afflicted with Alzheimer's Disease, a Multiple Sclerosis, a myocardial infarction, an atherosclerotic vascular disease, a microvasculopathy, a thyroiditis, an inflammatory bowel disease, an organ graft rejection, a membranous nephritis, a sympathetic ophthalmia, uveitis, or a sarcoidosis.
40. The method according to clause 39, wherein the positive results for expression or activity of at least one inflammasome activity marker in the inflammation-affected subject is elevated expression or activity of at least one inflammasome activity marker in comparison to expression or activity of at least one inflammasome activity marker in a subject not diagnosed with uveitis.
41. The method according to any one of clauses 33 to 40, wherein the positive results for expression or activity of at least one inflammasome activity marker in the inflammation-affected subject is determined by a histological score.
42. A method for inhibiting inflammasome activation in cells of an inflammation-affected subject, the method comprising: administering to the subject a composition comprising a nucleotide sequence encoding a membrane independent CD59 protein operably linked to a promoter for expression and secretion of the membrane independent CD59 protein in the cells of the inflammation-affected subject, wherein the composition inhibits inflammasome activation, wherein the subject has displayed positive results for expression or activity of at least one inflammasome activity marker.
43. The method according to clause 42, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.
44. The method according to clause 42, wherein the inflammasome activity marker is selected from: apoptosis-associated speck-like protein containing a CARD (PYCARD/ASC), or a NACHT, LRR and PYD domains-containing protein (NALP).
45. The method according to clause 44, wherein the NALP is NACHT, LRR and PYD domains-containing protein 3 (NLRP3).
46. The method according to any one of clauses 42 to 45, wherein the subject has displayed positive results for expression or activity of at least one inflammasome activity marker in an eye of the subject.
47. The method according to any one of clauses 42 to 46, further comprising measuring in the inflammation affected subject an inflammasome activity marker after the administering of the composition comprising the nucleotide sequence encoding the membrane independent CD59 protein operably linked to the promoter for the expression and secretion of the membrane independent CD59 protein.
48. The method according to any one of clauses 42 to 47, wherein the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.
49. The method according to clause 48, wherein the subject has been diagnosed with or is suspected of being afflicted with a uveitis.
50. The method according to any one of clauses 42 to 49, wherein the positive results for expression or activity of at least one inflammasome activity marker in the inflammation-affected subject is elevated expression or activity of at least one inflammasome activity marker in comparison to expression or activity of at least one inflammasome activity marker in an individual not diagnosed or afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a polyarteritis nodosa, a Wegener's granulomatosis a Vogt-Koyanagi-Harada Disease, a sympathetic ophthalmia, or a sarcoidosis.
51. The method according to clause 50, wherein the positive results for expression or activity of at least one inflammasome activity marker in the inflammation-affected cells of the subject is elevated expression or activity of at least one inflammasome activity marker in comparison to expression or activity of at least one inflammasome activity marker in an individual not diagnosed or afflicted with a uveitis.
52. The method according to any one of clauses 42 to 51, wherein the positive results for expression or activity of at least one inflammasome activity marker in the inflammation-affected subject is determined by a histological score.

## Claims

1. A gene therapy composition for use in inhibiting inflammasome activation in cells of an inflammation-affected eye in a subject afflicted with autoimmune uveitis, the composition comprising a viral expression vector encoding a membrane independent CD59 protein operably linked to a promoter for expression and secretion of the membrane independent CD59 protein in the cells of the inflammation-affected eye, wherein the composition inhibits inflammasome activation, and wherein the subject has displayed positive results for expression or activity of at least one inflammasome activity marker in the inflammation-affected eye of the subject.

2. The composition for use according to claim 1, wherein an inflammasome activity marker is measured after the administering of said composition.

3. The composition for use according to claims 1 or 2, wherein the positive results for expression or activity of at least one inflammasome activity marker in the inflammation-affected eye of the subject is elevated expression or activity of at least one inflammasome activity marker in comparison to expression or activity of at least one inflammasome activity marker in the eye of an individual not diagnosed or afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a polyarteritis nodosa, a Wegener's granulomatosis a Vogt-Koyanagi-Harada Disease, a sympathetic ophthalmia, or a sarcoidosis.

4. A gene therapy composition for use in inhibiting inflammasome activation in cells of an inflammation-affected subject afflicted with autoimmune uveitis comprising
a viral expression vector encoding a membrane independent CD59 protein operably linked to a promoter for expression and secretion of the membrane independent CD59 protein in the cells of the inflammation-affected subject; wherein the composition inhibits inflammasome activation; and wherein an inflammasome activity marker is measured in the subject, and if the inflammasome activity marker is positive, the composition is administered to the subject.

5. The composition for use according to claim 4, wherein the inflammasome activity marker is measured in an eye of the subject.

6. The composition for use according to claims 4 or 5, wherein an inflammasome activity marker is measured in the subject after the administering of said composition.

7. A gene therapy composition for use in inhibiting inflammasome activation in cells of an inflammation-affected subject afflicted with autoimmune uveitis comprising
a viral expression vector encoding a membrane independent CD59 protein operably linked to a promoter for expression and secretion of the membrane independent CD59 protein in the cells of the inflammation-affected subject,
wherein the composition inhibits inflammasome activation, and
wherein the subject has displayed positive results for expression or activity of at least one inflammasome activity marker in the inflammation-affected subject.

8. The composition for use according to claim 7, wherein the positive results for expression or activity of at least one inflammasome activity marker in the inflammation-affected subject is in an eye of the inflammation-affected subject.

9. A gene therapy composition for use in inhibiting inflammasome activation in cells of an autoimmune uveitis afflicted subject comprising a viral expression vector encoding a membrane independent CD59 protein operably linked to a promoter for expression and secretion of the membrane independent CD59 protein in the cells of the inflammation-affected subject, wherein the composition inhibits inflammasome activation.

10. The composition for use according to claim 9, wherein the subject has displayed positive results for expression or activity of at least one inflammasome activity marker in an eye of the inflammation-affected subject.

11. A gene therapy composition for use in inhibiting inflammasome activation in cells of an inflammation-affected subject afflicted with autoimmune uveitis, the composition comprising a viral expression vector encoding a membrane independent CD59 protein operably linked to a promoter for expression and secretion of the membrane independent CD59 protein in the cells of the inflammation-affected subject, wherein the composition inhibits inflammasome activation, wherein the subject has displayed positive results for expression or activity of at least one inflammasome activity marker.

12. The composition for use according to claim 11, wherein the subject has displayed positive results for expression or activity of at least one inflammasome activity marker in an eye of the subject.

13. The composition for use according to claim 11 or 12, further comprising measuring in the inflammation affected subject an inflammasome activity marker after the administering of said composition.

14. The composition for use according to any one of claims 1 to 13, wherein the inflammasome activity marker is selected from: caspase 1, caspase 5, IL-1β, IL-β17, IL-18, apoptosis-associated speck-like protein containing a CARD (PYCARD/ASC), a NACHT, LRR and PYD domains-containing protein (NALP), IFN-γ, a Th1 T-cell marker or cytokine, a Th17 T-cell marker or cytokine, and CD4+.

15. The composition for use according to any one of claims 1 to 14, wherein the subject has been diagnosed with or is suspected of being afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a systemic lupus erythematosus, a polyarteritis nodosa, a Wegener's granulomatosis a Vogt-Koyanagi-Harada Disease, a sympathetic ophthalmia, and a sarcoidosis.

16. The composition for use according to any one of claims 1 to 15, wherein the positive inflammasome activity marker or positive results for expression or activity of at least one inflammasome activity marker in the inflammation-affected subject is elevated expression or activity of at least one inflammasome activity marker in comparison to expression or activity of at least one inflammasome activity marker in an individual not diagnosed or afflicted with a uveitis, an allergic conjunctivitis, a blepharitis, a chronic conjunctivitis, an episcleritis, a keratitis, a retinitis, an ocular cicatricial pemphigoid, a mucous membrane pemphigoid, a pterygium scleritis, a Stevens-Johnson syndrome, an Eales Disease, a Behcet's disease, a sarcoidosis, a polyarteritis nodosa, a Wegener's granulomatosis a Vogt-Koyanagi-Harada Disease, a sympathetic ophthalmia, or a sarcoidosis.

17. The composition for use according to any one of claims 1 to 16, wherein the viral expression vector is at least one selected from the group of: an adenovirus, a lentivirus, an adeno-associated virus (AAV), a helper-dependent adenovirus, and a herpesvirus.

18. The composition for use according to any one of claims 1 to 17, wherein the viral expression vector is an AAV serotype 2 (AAV2).
